# FASCICULE DE BREVET EUROPEEN

(11) **EP 1 768 985 B1**
(45) Date de publication et mention de la délivrance du brevet: **23.01.2008**
(21) Numéro de dépôt: 05778708.7
(22) Date de dépôt: 22.06.2005
(51) Int. Cl.: C07D 495/14, C07D 487/04, C07D 471/14, C07D 495/22

(54) **DERIVES DE PYRIMIDO-BENZIMIDAZOLE ET LEUR UTILISATION COMME AGONISTES OU ANTGONISTES DES RÉCEPTEURS DES MÉLANOCORTINES**
PYRIMDOBENZIMIDAZOLDERIVATE UND VERWENDUNG DAMIT ALS AGONISTEN ODER ANTAGONISTEN VON MELANOCORTINREZEPTOREN
PYRIMIDO-BENZIMIDAZOLE DERIVATIVES AND THE USE THEREOF IN THE FORM OF AGONISTS OR ANTAGONISTS OF MELANOCORTIN RECEPTORS

(30) Priorité: 24.06.2004 FR 0406903
(43) Date de publication de la demande: 04.04.2007
(73) Titulaire: Société de Conseils de Recherches et d'Applications Scientifiques ( S.C.R.A.S.), 75016 Paris (FR)
(72) Inventeur: POITOUT, Lydie, F-92160 Antony (FR); SACKUR, Carole, F-75011 Paris (FR); BRAULT, Valérie, F-78730 Saint-Arnoult-en-Yvelines (FR); ROUBERT, Pierre, F-75014 Paris (FR); PLAS, Pascale, F-92320 Châtillon (FR)
(74) Mandataire: Bourgouin, André
(86) Numéro de dépôt international: PCT/FR2005/001563
(87) Numéro de publication internationale: WO 2006/010811

(56) Documents cités:
- EP-A- 0 311 321
- US-A- 4 376 121
- US-A- 5 625 063
- US-A1- 2002 004 512

## Description

La présente demande a pour objet de nouveaux dérivés pyrimido-benzimidazole. Ces produits ont une bonne affinité pour certains sous-types de récepteurs des mélanocortines, en particulier des récepteurs MC4. Ils sont particulièrement intéressants pour traiter les états pathologiques et les maladies dans lesquels un ou plusieurs récepteurs des mélanocortines sont impliqués. L'invention concerne également des compositions pharmaceutiques contenant lesdits produits et leur utilisation pour la préparation d'un médicament.

Les mélanocortines représentent un groupe de peptides qui dérivent d'un même précurseur, la proopiomélanocortine (POMC), et qui sont structurellement proches : L'hormone adrénocorticotrope (ACTH), l'hormone stimulante des mélanocytes α (α-MSH), la β-MSH et la γ-MSH (Eipper B.A. et Mains R.E., Endocr. Rev. 1980, 1, 1-27). Les mélanocortines exercent de nombreuses fonctions physiologiques. Elles stimulent la synthèse des stéroïdes par la cortico-surrénale et la synthèse d'eumélanine par les mélanocytes. Elles régulent la prise de nourriture, le métabolisme énergétique, la fonction sexuelle, la régénération neuronale, la pression sanguine et la fréquence cardiaque, ainsi que la perception de la douleur, l'apprentissage, l'attention et la mémoire. Les mélanocortines possèdent également des propriétés anti-inflammatoires et anti-pyrétiques et contrôlent la sécrétion de plusieurs glandes endocrines ou exocrines telles les glandes sébacées, lacrymales, mammaires, la prostate et le pancréas (Wikberg J.E. et al. Pharmacol. Res. 2000, 42, 393-420 ; Abdel-Malelc Z.A., Cell. Mol. Life. Sci. 2001, 58, 434-441).

Les effets des mélanocortines sont médiés par une famille de récepteurs membranaires spécifiques à sept domaines transmembranaires et couplés aux protéines G. Cinq sous-types de récepteurs, nommés MC1 à MC5, ont été clonés et caractérisés à ce jour. Ces récepteurs diffèrent par leur distribution tissulaire et par l'affinité des différentes mélanocortines, les récepteurs MC2 ne reconnaissant que l'ACTH. La stimulation des récepteurs des mélanocortines active l'adénylate cyclase avec production d'AMP cyclique. Si les rôles fonctionnels spécifiques de chacun des récepteurs ne sont pas totalement élucidés, le traitement de désordres pathologiques ou de maladies peut être associé à une affinité pour certains sous-types de récepteurs. Ainsi l'activation des récepteurs MC 1 a été associée au traitement des inflammations, alors que leur blocage a été associé au traitement de cancers cutanés. Le traitement des troubles de la nutrition a été associé aux récepteurs MC3 et MC4, le traitement de l'obésité par les agonistes et le traitement de la cachexie et de l'anorexie par les antagonistes. D'autres indications associées à l'activation des récepteurs MC3 et MC4 sont les troubles de l'activité sexuelle, les douleurs neuropathiques, l'anxiété, la dépression et les toxicomanies. L'activation des récepteurs MC5 a été associée au traitement de l'acné et des dermatoses.

Les déposants ont trouvé que les nouveaux composés de formule générale (I) décrits ci-après possèdent une bonne affinité pour les récepteurs des mélanocortines. Ils agissent préférentiellement sur les récepteurs MC4. Lesdits composés, agonistes ou antagonistes des récepteurs des mélanocortines, peuvent être utilisés pour traiter les états pathologiques ou les maladies métaboliques, du système nerveux ou dermatologiques, dans lesquels un ou plusieurs récepteurs des mélanocortines sont impliqués tels que les exemples suivants : les états inflammatoires, les troubles de l'homéostasie énergétique, de la prise de nourriture, les désordres pondéraux (l'obésité, la cachexie, l'anorexie), les désordres de l'activité sexuelle (les troubles de l'érection), la douleur et plus particulièrement la douleur neuropathique. On peut également citer les troubles mentaux (l'anxiété, la dépression), les toxicomanies, les maladies de la peau (l'acné, les dermatoses, les cancers cutanés, les mélanomes). Ces composés peuvent également être utilisés pour stimuler la régénération nerveuse.

L'invention a donc pour objet des composés de formule générale (I) 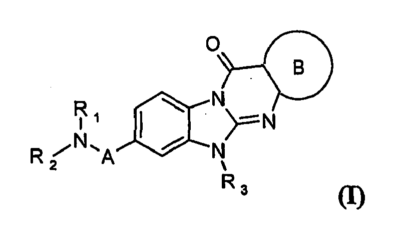 sous forme racémique, d'énantiomère ou toutes combinaisons de ces formes et dans laquelle :
A représente -CH₂- ou -C(O)- ;
R₁ représente l'atome d'hydrogène ; un radical (C₁-C₈)alkyle éventuellement substitué par hydroxy ou un ou plusieurs radicaux halo identiques ou différents ; (C₂-C₆)alkényle ; ou un radical de formule -(CH₂)ₙ-X₁
R₂ représente un radical (C₁-C₈)alkyle éventuellement substitué par hydroxy ou un ou plusieurs radicaux halo identiques ou différents; (C₂-C₆)alkényle-; ou un radical de formule -(CH₂)ₙ-X₁ ;
chaque X₁ représente, indépendamment, un radical (C₁-C₆)alkoxy, (C₃-C₇)cycloalkyle, adamantyle, hétérocycloalkyle, aryle ou hétéroaryle,
   les radicaux (C₃-C₇)cycloalkyle, hétérocycloalkyle, aryle et hétéroaryle étant éventuellement substitués par un ou plusieurs substituants identiques ou différents choisis parmi : -(CH₂)_{n'}-V₁-Y₁, halo, nitro, cyano et aryle ;
   V₁ représente -O-, -S- ou une liaison covalente ;
   Y₁ représente un radical (C₁-C₆)alkyle éventuellement substitué par un ou plusieurs radicaux halo identiques ou différents ;
   n représente un entier de 0 à 6 et n' un entier de 0 à 2 (étant entendu que lorsque n est égal à 0, alors X₁ ne représente pas le radical alkoxy) ;
ou bien R₁ et R₂ forment ensemble, avec l'atome d'azote auquel ils sont rattachés,
   - un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)-NV₁'Y₁' avec V₁' et Y₁' représentant, indépendamment, l'atome d'hydrogène ou un (C₁-C₆)alkyle ; ou
   - un radical de formule : 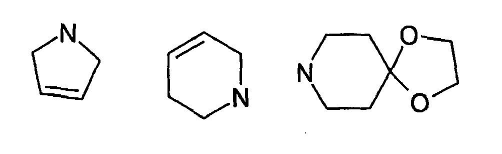
R₃ représente un radical de formule-(CH₂)ₛ-R'₃ ;
R'₃ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₃W'₃
   W₃ représenté l'atome d'hydrogène ou (C₁-C₈)alkyle;
   W'₃ représente un radical de formule -(CH₂)_{s'}-Z₃ ;
   Z₃ représente l'atome d'hydrogène, (C₁-C₈)alkyle éventuellement substitué par un ou plusieurs substituants identiques ou différents choisis parmi : (C₁-C₆)alkoxy, (C₁-C₆)alkylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
      les radicaux aryle et hétéroaryle étant éventuellement substitués par un ou plusieurs radicaux identiques ou différents choisis parmi : le radical de formule -(CH₂)_{s"}-V₃-Y₃, hydroxy, halo, nitro et cyano ;
         V₃ représente -O-, -S-, -NH-C(O)-, -NV'₃ ou une liaison covalente ;
         Y₃ représente un atome d'hydrogène ou un radical (C₁-C₆)alkyle éventuellement substitué par un ou plusieurs radicaux halo identiques ou différents ;
         V₃' représente un atome d'hydrogène ou un (C₁-C₆)allcyle ;
         s" représente un entier de 0 à 4 ;
   ou bien Z₃ représente un radical de formule 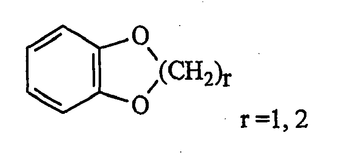
   s et s'représentent, indépendamment, un entier de 0 à 6 ;
B représente un radical mono- ou bi-cyclique condensé, insaturé, aromatique ou non-aromatique, contenant éventuellement un ou plusieurs hétéroatomes identiques ou différents choisis parmi O, S et N, et éventuellement substitué par un ou plusieurs radicaux, identiques ou différents, choisis parmi : halo, nitro, cyano, oxy, -X_{B}-Y_{B}, et aryle éventuellement substitué par un ou plusieurs substituants choisis parmi : halo et (C₁-C₆)alkyle éventuellement substitué par un ou plusieurs radicaux halo identiques ou différents ;
   X_{B} représente une liaison covalente, -O-, -S-, -C(O)-, -NR_{N}-C(O)-, -C(O)-NR_{N}-, -C(O)-O-, -SO₂- ou -SO₂NH- ;
   Y_{B} représente l'atome d'hydrogène ou un radical (C₁-C₆)alkyle éventuellement substitué par un ou plusieurs radicaux halo identiques ou différents ;
   R_{N} représente l'atome d'hydrogène ou un radical (C₁-C₆)alkyle ;
ou un sel pharmaceutiquement acceptable de ces derniers.

Dans les définitions indiquées ci-dessus, l'expression halo représente le radical fluoro, chloro, bromo ou iodo, de préférence chloro, fluoro ou bromo. L'expression alkyle (lorsqu'il n'est pas donné plus de précision), représente de préférence un radical alkyle ayant de 1 à 6 atomes de carbone, linéaire ou ramifié, tels que les radicaux méthyle, éthyle, propyle, isopropyle, butyle, isobutyle, sec-butyle et *tert*-butyle, pentyle ou amyle, isopentyle, néopentyle, 2,2-diméthyl-propyle, hexyle, isohexyle ou 1,2,2-triméthyl-propyle. Le terme (C₁-C₈)alkyle désigne un radical alkyle ayant de 1 à 8 atomes de carbone, linéaire ou ramifié, tels les radicaux contenant de 1 à 6 atomes de carbone tels que définis ci-dessus mais également heptyle, octyle, 1,1,2,2-tétraméthylpropyle, 1,1,3,3-tétraméthyl-butyle. Par l'expression alkyle substitué par hydroxy, il faut comprendre toute chaîne alkyle linéaire ou ramifiée, contenant un radical hydroxy positionnée le long de la chaîne ; ainsi pour une chaîne contenant 3 atomes de carbone et un radical hydroxy, on peut donner comme exemples HO-(CH₂)₃-, CH₃-CH(OH)-CH₂- et CH₃-CH₂-CH(OH)-.

Par alkényle, lorsqu'il n'est pas donné plus de précision, on entend un radical alkyle linéaire ou ramifié comptant de 2 à 6 atomes de carbone et présentant au moins une insaturation (double liaison), comme par exemple vinyle, allyle, propényle, butènyle ou pentènyle.

Le terme alkoxy désigne les radicaux dans lesquels le radical alkyle est tel que défini ci-dessus comme par exemple les radicaux méthoxy, éthoxy, propyloxy ou isopropyloxy mais également butoxy linéaire, secondaire ou tertiaire, pentyloxy. Le terme alkoxy-carbonyle désigne de préférence les radicaux dans lesquels le radical alkoxy est tel que défini ci-dessus comme par exemple méthoxycarbonyle, éthoxycarbonyle. Le terme alkylthio désigne les radicaux dans lesquels le radical alkyle est tel que défini ci-dessus comme par exemple méthylthio, éthylthio. Le terme guanidino représente le radical -NHC(=NH)NH₂.

Le terme (C₃-C₇)cycloalkyle désigne un système monocyclique carboné saturé comprenant de 3 à 7 atomes de carbone, et de préférence les cycles cyclopropyle, cyclobutyle, cyclopentyle, cyclohexyle ou cycloheptyle. L'expression hétérocycloalkyle désigne un système saturé monocyclique ou bicyclique condensé contenant de 2 à 9 atomes de carbone et au moins un hétéroatome. Ce radical peut contenir plusieurs hétéroatomes identiques ou différents. De préférence, les hétéroatomes sont choisis parmi l'oxygène, le soufre ou l'azote. Comme exemple d'hétérocycloalkyle, on peut citer : les cycles de 5 ou 6 chaînons contenant au moins un atome d'azote tels que pyrrolidine, imidazolidine, pyrrazolidine, isothiazolidine, thiazolidine, isoxazolidine, oxazolidine, pipéridine, pipérazine, morpholine, les cycles de plus de 6 chaînons et contenant au moins un atome d'azote tels que azépane (azacycloheptane), azacyclooctane, diazépane, décahydroisoquinoline (ou décahydroquinoline), mais également les cycles ne contenant aucun atome d'azote tels que tétrahydrofurane (radical tétrahydrofuryle) ou tétrahydrothiophène (radical tétrahydrothiényle).

Le terme hétérobicycloalkyle désigne un système bicyclique hydrocarboné saturé non condensé contenant de 5 à 8 atomes de carbone et au moins un hétéroatome choisi parmi l'azote, l'oxygène et le soufre. Comme exemple d'hétérobicycloalkyle, on peut citer les aza-bicycloheptane et aza-bicyclooctane tels que 7-aza-bicyclo[2,2,1]heptane, 2-aza-bicyclo[2,2,2]octane ou 6-aza-bicyclo[3,2,1]octane.

L'expression aryle représente un radical aromatique, constitué d'un cycle ou de cycles condensés, comme par exemple le radical phényle, naphtyle, fluorényle ou anthryle.

L'expression hétéroaryle désigne un radical aromatique, constitué d'un cycle ou de cycles condensés, avec au moins un cycle contenant un ou plusieurs hétéroatomes identiques ou différents choisis parmi le soufre, l'azote ou l'oxygène. Comme exemple de radical hétéroaryle, on peut citer les radicaux contenant au moins un atome d'azote tels que pyrrolyle, imidazolyle, pyrazolyle, isothiazolyle, thiazolyle, isoxazolyle, oxazolyle, oxadiazolyle, triazolyle, thiadiazolyle, pyridyle, pyrazinyle, pyrimidyle, pyridazinyle, quinolyle, isoquinolyle, quinoxalinyle, indolyle, benzoxadiazoyle, carbazolyle, phénoxazinyle, thiéno-pyridinyle (thiéno[2,3-b]pyridine, thiéno[3,2-b]pyridine, thiéno[2,3-c]pyridine, thiéno[3,2-c]pyridine, thiéno[3,4-b]pyridine, thiéno[3,4-c]pyridine), thiéno-pyrazinyle (thiéno[2,3-b]pyrazine, thiéno[3,4-b]pyrazine) mais également les radicaux ne contenant pas d'atome d'azote tels que thiényle, benzothiényle, furyle, benzofuryle, dibenzofuryle, dihydrobenzofuryle, dibenzothiènyle, thioxanthènyle, ou pyranyle.

Le terme aralkyle (arylalkyle) désigne de préférence les radicaux dans lesquels les radicaux aryle et alkyle sont tels que définis ci-dessus comme par exemple benzyle ou phénéthyle.

L'expression radical mono- ou bi-cyclique condensé, insaturé, aromatique, peut être illustrée soit par le radical aryle tel que défini ci-dessus lorsque ledit radical aromatique ne contient pas d'hétéroatome, soit par le radical hétéroaryle tel que défini ci-dessus lorsque ledit radical aromatique contient au moins un hétéroatome.

L'expression radical mono- ou bi-cyclique condensé, insaturé, non-aromatique, et ne contenant aucun hétéroatome, peut être illustrée par cyclopentenyle ou cyclohexenyle.

L'expression radical mono- ou bi-cyclique condensé, insaturé, non-aromatique et contenant au moins un hétéroatome, peut être illustrée par les radicaux hétéroaryle tels que définis ci-dessus et dans lesquels au moins une double liaison est hydrogénée. On peut ainsi citer comme exemple les radicaux associés aux cycles suivants : dihydroindolyle, dihydrothiophène (2,5-dihydrothiophène, 2,3-dihydrothiophène), tétrahydropyridine (2,3,4,5-tétrahydropyridine, 1,2,3,6-tétrahydropyridine, 1,2,3,4-tétrahydropyridine), tétrahydro-thiéno-pyridine (4,5,6,7-tétrahydro-thiéno[3,2-b]pyridine, 4,5,6,7-tétrahydro-thiéno[2,3-c]pyridine, 4,5,6,7-tétrahydro-thiéno[3,2-c]pyridine), tétrahydropyrimidine (2,3,4,5-tétrahydropyrimidine, 1,2,3,4-tétrahydropyrimidine, 1,4,5,6-tétrahydropyrimidine), tétrahydrobenzothiophène (4,5,6,7-tétrahydro-1-benzothiophène), dihydrocyclopentathiophène (5,6-dihydro-4H-cyclopenta[b]thiophène, benzodioxole, dihydro-benzodioxine.

Dans la présente demande également, le radical -(CH₂); (i entier pouvant représenter n, n', s, s' et s" tels que définis ci-dessus), représente une chaîne hydrocarbonée, linéaire ou ramifiée, de i atomes de carbone. Ainsi le radical -(CH₂)₃- peut représenter -CH₂-CH₂-.

CH₂- mais également -CH(CH₃)-CH₂-, -CH₂-CH(CH₃)- ou -C(CH₃)₂-.

Selon la présente demande également, lorsqu'un radical a pour formule -B-D-E avec D représentant par exemple -C(O)-NH-, cela signifie que l'atome de carbone de -C(O)-NH- est lié à B et l'atome d'azote à E.

De préférence, l'invention concerne des composés de formule I telle que définie ci-dessus et caractérisés en ce que A représente -C(O)- ; ou un sel pharmaceutiquement acceptable de ces derniers.

De préférence également, l'invention concerne des composés de formule I telle que définie ci-dessus, caractérisés en ce que R₁ et R₂ représentent, indépendamment, un radical (C₁-C₈)alkyle ; ou un sel pharmaceutiquement acceptable de ces derniers.

De manière très préférentielle, l'invention concerne des composés de formule I telle que définie ci-dessus, caractérisés en ce que R₁ et R₂ représentent, indépendamment, un radical (C₁-C₆)alkyle, et plus particulièrement R₁ et R₂ représentent, indépendamment, un radical butyle, pentyle ou isopentyle ; ou un sel pharmaceutiquement acceptable de ces derniers.

De préférence également, l'invention concerne des composés de formule 1 telle que définie ci-dessus, caractérisés en ce que R'₃ représente un hétérocycloalkyle contenant au moins un atome d'azote et éventuellement substitué par (C₁-C₆)alkyle ou benzyle ; ou un radical de formule -NW₃W'₃ dans laquelle W₃ représente l'atome d'hydrogène ou un radical (C₁-C₈)alkyle, et W'₃ le radical Z₃ et Z₃ représente l'atome d'hydrogène ou un radical (C₁-C₈)alkyle ; ou un sel pharmaceutiquement acceptable de ces derniers.

De préférence également, l'invention concerne des composés de formule 1 telle que définie ci-dessus, caractérisés en ce que B représente un radical choisi parmi : phényle, thiényle, pyrrolyle, pyrazolyle, pyridyle, pyrimidyle, pyrazinyle, benzothiényle, thiéno-pyridinyle, thiéno-pyrazinyle, indolyle, benzofuryle, cyclopentènyle, cyclohexenyle, 1,2,3,6-tétrahydropyridinyle, 1,2,3,4-tétrahydropyrimidinyle, 4,5,6,7-tétrahydro-thiéno[2,3-c]pyridinyle, tétrahydrobenzothiényle et dihydrocyclopentathiényle.

De préférence également, l'invention concerne des composés de formule I telle que définie ci-dessus, caractérisés en ce que B est éventuellement substitué par un ou plusieurs radicaux, identiques ou différents, choisis parmi : halo, nitro, cyano, oxy, -X_{B}-Y_{B}, et phényle éventuellement substitué par un ou plusieurs substituants choisis parmi : halo et (C₁-C₆)alkyle éventuellement substitué par un ou plusieurs radicaux halo identiques ou différents ;
X_{B} représente une liaison covalente, -O-, -S-, -C(O)-, -NR_{N}-C(O)- ou -C(O)-O- ;
Y_{B} représente l'atome d'hydrogène ou un radical (C₁-C₆)alkyle éventuellement substitué par un ou plusieurs radicaux halo identiques ou différents ;
R_{N} représente l'atome d'hydrogène ; ou un sel pharmaceutiquement acceptable de ces derniers.

De préférence également, l'invention concerne des composés de formule I telle que définie ci-dessus, caractérisés en ce que B représente un radical choisi parmi : phényle, furyle, thiényle, pyrrolyle, pyrazolyle, pyridyle, thiazolyle, pyrazinyle, benzothiényle, thiéno-pyridinyle, thiéno-pyrazinyle, indolyle, benzofuryle, cyclohexenyle, 1,2,3,6-tétrahydropyridinyle, tétrahydrobenzothiényle et dihydrocyclopentathiényle.

De préférence également, l'invention concerne des composés de formule I telle que définie ci-dessus, caractérisés en ce que B est éventuellement substitué par un ou plusieurs radicaux, identiques ou différents, choisis parmi : halo, nitro, cyano, -X_{B}-Y_{B}, et phényle éventuellement substitué par un ou plusieurs substituants choisis parmi : halo et (C₁ -C₆)alkyle ;
X_{B} représente une liaison covalente, -O-, -S-, -C(O)- ou -C(O)-O- ;
Y_{B} représente l'atome d'hydrogène ou un radical (C₁-C₆)alkyle ; ou un sel pharmaceutiquement acceptable de ces derniers.

De préférence également, l'invention concerne des composés de formule I telle que définie ci-dessus, caractérisés en ce que B représente un radical choisi parmi : phényle, furyle, thiényle, pyrrolyle, pyrazolyle, pyridyle, pyrazinyle, benzothiényle, thiéno-pyridinyle, indolyle, benzofuryle, cyclohexenyle, 1,2,3,6-tétrahydropyridinyle, tétrahydrobenzothiényle et dihydrocyclopentathiényle.

De préférence également, l'invention concerne des composés de formule I telle que définie ci-dessus, caractérisés en ce que B est éventuellement substitué par un ou plusieurs radicaux, identiques ou différents, choisis parmi : halo, nitro et -X_{B}-Y_{B} ;
X_{B} représente une liaison covalente, -O-, -C(O)- ou -C(O)-O- ;
Y_{B} représente un radical (C₁-C₆)alkyle ;
et de manière très préférentielle B est éventuellement substitué par un ou plusieurs radicaux, identiques ou différents, choisis parmi : halo, nitro ou -X_{B}-Y_{B} ; X_{B} représente une liaison covalente ou -O- et Y_{B} représente un radical (C₁-C₆)alkyle ; ou un sel pharmaceutiquement acceptable de ces derniers.

De manière très préférentielle, l'invention concerne des composés de formule I telle que définie ci-dessus, caractérisés en ce que R'₃ représente un hétérocycloalkyle contenant au moins un atome d'azote et éventuellement substitué par (C₁-C₆)alkyle, et en particulier le radical pipéridinyle ou pyrrolidinyle ; ou un sel pharmaceutiquement acceptable de ces derniers.

De préférence également, l'invention concerne des composés de formule I telle que définie ci-dessus, caractérisés en ce que R'₃ représente un hétérocycloalkyle de 5 à 6 chaînons et contenant un seul atome d'azote et éventuellement un atome d'oxygène ; ou un radical de formule -NW₃W'₃ dans laquelle W₃ représente l'atome d'hydrogène ou un radical (C₁-C₆)alkyle, W'₃ représente le radical Z₃ et Z₃ représente l'atome d'hydrogène ou un radical (C₁-C₆)alkyle ; ou un sel pharmaceutiquement acceptable de ces derniers.

De manière très préférentielle également, l'invention concerne des composés de formule I telle que définie ci-dessus, caractérisés en ce que R'₃ représente le radical pipéridinyle ou pyrrolidinyle, et s représente un entier de 2 à 4 ; ou un sel pharmaceutiquement acceptable de ces derniers.

De manière très préférentielle également, l'invention concerne des composés de formule I telle que définie ci-dessus, caractérisés en ce que B représente un radical choisi parmi : phényle, thiényle, pyrrolyle, pyrazolyle, pyridyle, pyrazinyle, benzothiényle, thiéno-pyridinyle, thiéno-pyrazinyle, indolyle, benzofuryle, cyclohexenyle, 4,5,6,7-tétrahydro-thiéno[2,3-c]pyridinyle et dihydrocyclopentathiényle.

Suivant les définitions des groupes variables A, B, R₁, R₂ et R₃, les composés selon l'invention peuvent être préparés en phase liquide selon les différentes procédures A à C décrites ci-dessous.

### A. Préparation selon le schéma réactionnel A:

Les composés de formule (I) selon l'invention dans laquelle A représente -C(O)-, peuvent être préparés selon le schéma A suivant :

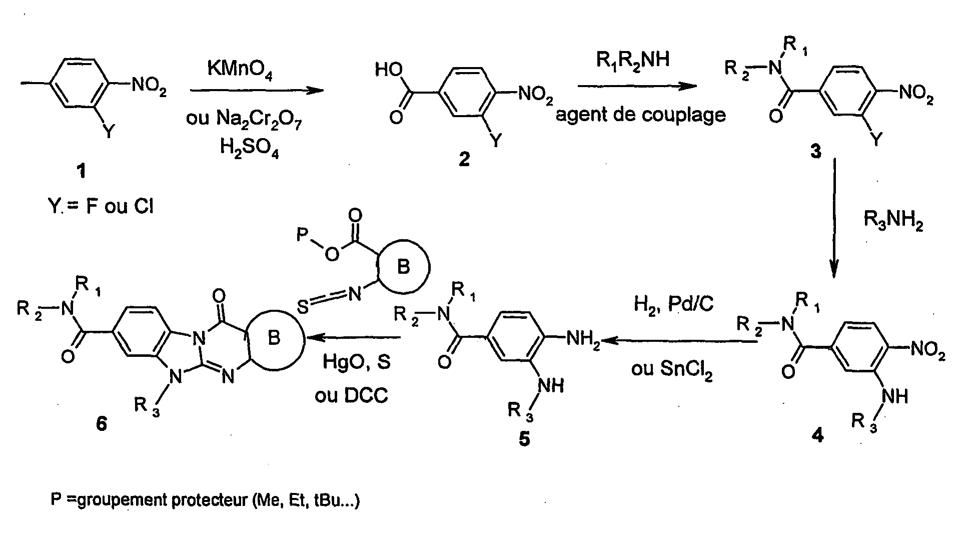

Comme décrit dans le schéma A, le dérivé méthylé (1) peut être oxydé en acide carboxylique (2) par une solution aqueuse de permanganate de potassium à une température de 100° C pendant 3 à 6 heures (selon procédure de Schmelkes *et al, J. Am. Chem. Soc,* **1944**, 1631), ou par une solution aqueuse de dichromate de sodium en présence d'acide sulfurique à une température de 20-90° C pendant 1 à 3 heures (selon procédure de Howes et al, European J. Med. Chem, 1999, 34, 225-234). L'acide carboxylique (2) peut être couplé avec une amine primaire ou secondaire en présence d'un agent de couplage tel que le diisopropylcarbodiimide (DIC), le dicyclohexylcarbodiimide (DCC), le chlorhydrate de 1-3-dimethylaminopropyl)-3-éthylcarbodiimide (EDC) ou le carbonyldiimidazole (CDI) avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (3). Le traitement du dérivé fluoré ou chloré (3) par une amine primaire en présence d'une base inorganique telle que du carbonate de césium ou de potassium dans un solvant organique inerte tel que le diméthylformamide ou l'acétonitrile à une température de 20-100° C pendant 2 à 48 heures conduit au dérivé (4). La fonction nitro du composé (4) est réduite par traitement avec du chlorure d'étain dihydrate dans un solvant inerte tel que l'acétate d'éthyle ou le diméthylformamide à une température de 60-80° C pendant 3 à 15 heures, ou par hydrogénation catalytique en présence de palladium sur charbon 10 % dans un solvant inerte tel que le méthanol, éthanol, acétate d'éthyle ou un mélange de ces solvants, à une température de 18-25°C, pendant 2 à 8 heures pour conduire à la dianiline (5). Le dérivé (5) est ensuite traité par un isothiocyanate ortho-ester en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène, en présence ou non d'une base organique telle que la triéthylamine, diisopropyléthylamine, méthylate, éthylate ou *tert*-butylate de sodium, dans un solvant inerte tel que le tétrahydrofuranne, chlorure de méthylène, ou chloroforme à une température de 20-70° C pendant 2 à 72 heures, ou sous micro-ondes (équipement Personal Chemistry^{®}), dans un tube fermé, à 100° C pendant 10 à 30 minutes pour conduire au dérivé (6). Alternativement, le dérivé (5) peut être traité par un isothiocyanate ortho-ester en présence d'oxyde de mercure (II) jaune et d'une quantité catalytique de soufre, en présence ou non d'une base organique telle que la triéthylamine, diisopropyléthylamine, méthylate, éthylate ou *tert*-butylate de sodium, dans un solvant polaire tel que le méthanol ou l'éthanol pendant 2 à 24 heures à une température de 20-80° C, ou sous micro-ondes (équipement Personal Chemistry^{®}), dans un tube scellé, à 100° C pendant 10 à 30 minutes pour conduire à (6).

### Exemple A1 : chlorhydrate de N,N-bis(3-méthylbutyl)-11-oxo-5-(3-pipéridin-1-ylpropyl)-5,11-dihydrothiéno[3',2':4,5]pyrimido [1,2-a] benzimidazole-7-carboxamide

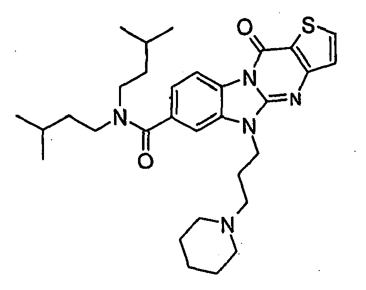

### Etape 1 : acide 3-fluoro-4-nitrobenzoïque

Un mélange de 3-fluoro-4-nitrotoluène (10 g, 1 eq) et de permanganate de potassium (25,5 g, 2,5 eq) dans l'eau (1 L) est chauffé au reflux pendant 6 heures puis refroidi à température ambiante. Le mélange est filtré sur célite et la phase aqueuse est lavée deux fois à l'éther diéthylique (2 x 300 ml). La phase aqueuse est acidifiée, à 0° C, avec une solution d'acide chlorhydrique concentrée puis concentrée sous pression réduite à 40° C jusqu'à un volume d'environ 300 ml. Le précipité formé est filtré puis lavé à l'éther de pétrole et séché pour donner le composé attendu sous forme d'un solide blanc (6,9 g ; 58 % rendement).

RMN (¹H, 400 MHz, DMSO-d₆) : δ 7,93 (m, 2H), 8,25 (m, 1H), 13,95 (m, 1H).

### Etape 2 : 3-fluoro-N,N-bis(3-méthylbutyl)-4-nitrobenzamide

A l'acide 3-fluoro-4-nitrobenzoïque (10 g, 1 eq) en solution dans le THF anhydre (60 ml) sont successivement additionnés le 1-hydroxybenzotriazole (HOBt) (8 g, 1,1 eq) en solution dans le THF (60 ml) et le chlorhydrate de 1-(3-diméthylaminopropyl)-3-éthylcarbodiimide (EDC) (11,4 g, 1,1 eq) en solution dans le chloroforme (60 ml). Le mélange est agité 2 heures à une température d'environ 20° C puis la diisoamylamine (12,2 ml, 1,1 eq) est additionnée. Après 4 heures d'agitation à une température d'environ 20° C, le mélange réactionnel est concentré sous pression réduite à 40° C. Le résidu est repris par du dichlorométhane (300 ml) et de l'eau (100 ml). Après décantation et extractions, les phases organiques combinées sont lavées avec de la saumure, séchées sur Na₂SO₄ puis concentrées sous pression réduite à 40° C. La purification par chromatographie éclair sur gel de silice (éluant : heptane 100 % à heptane/ acétate d'éthyle 70:30) donne le composé attendu (12,36 g ; 71 % rendement).

### SM/CL : MM calculée = 324,4 ; m/z = 325,2 (MH+)

RMN (¹H, 400 MHz, DMSO-d₆) : δ 0,69 (d, 6H), 0,93 (d, 6H), 1,35-1,60 (m, 6H), 3,09 (m, 2H), 3,41 (m, 2H), 7,38 (AB, 1H), 7,63 (AB, 1H), 8,21 (t, 1H).

### Etape 3 : N,N bis(3-méthylbutyl)-4-nitro-3-[(3-pipéridin-1-ylpropyl)amino]benzamide

Un mélange de 3-fluoro-N,N-bis(3-méthylbutyl)-4-nitrobenzamide (4 g, 1 eq), de 3-aminopropylpipéridine (1,9 g, 1,1 eq) et de carbonate de potassium (3,4 g, 2 eq) dans l'acétonitrile (150 ml) est chauffé au reflux pendant 3 heures puis concentré sous pression réduite à 40° C. Le résidu est repris par du dichlorométhane (200 ml) et de l'eau (80 ml). Après décantation et extractions, les phases organiques combinées sont lavées avec de la saumure, séchées sur Na₂SO₄ puis concentrées sous pression réduite à 40° C. La purification du résidu par chromatographie éclair sur gel de silice (éluant : acétate d'éthyle 100 % à acétate d'éthyle/méthanol 80:20) donne le composé attendu sous forme d'une huile jaune (5,5 g ; 100 % rendement).

SM/CL : MM calculée = 446,6 ; m/z = 447,3 (MH+)

RMN (¹H, 400 MHz, DMSO-d₆) : δ 0,68 (d, 6H), 0,92 (d, 6H), 1,31-1,69 (m, 12H), 1,74 (m, 2H), 2,32 (m, 6H), 3,10 (t, 2H), 3,38 (m, 4H), 6,53 (AB, 1H), 6,91 (s, 1H), 8,09 (d, 1H), 8,44 (t,1 H).

### Etape 4 : 4-amino-N,N-bis(3-méthylbutyl)-3-[(3-pipéridin-1-ylpropyl)amino]benzamide

Dans un autoclave sont additionnés le N,N-bis(3-méthylbutyl)-4-nitro-3-[(3-pipéridin-1-ylpropyl)amino]benzamide (1 g) en solution dans un mélange d'acétate d'éthyle / éthanol 2:1 (100 ml) et le palladium sur charbon 10 % (100 mg). Après 3 heures d'agitation sous atmosphère d'hydrogène (3 bars) à une température d'environ 20° C, le catalyseur est éliminé par filtration sur célite et le filtrat est concentré sous pression réduite à 40° C pour donner le composé attendu sous forme d'une huile (910 mg, 97 % rendement).

SM/CL : MM calculée = 416,6 ; m/z = 417,3 (MH+)

RMN (¹H, 400 MHz, DMSO-d₆) : δ 0,81 (d, 12H), 1,39-1,69 (m, 12H), 1,73 (m, 2H), 2,32 (m, 6H), 3,03 (m, 2H), 3,38 (m, 4H), 4,62 (s, 1H), 4,76 (s, 2H), 6,36 (s, 1H), 6,42 (AB, 1H), 6,50 (AB, 1H).

### Etape 5 : chlorhydrate de N,N-bis(3-méthylbutyl)-11-oxo-5-(3-pipéridin-1-ylpropyl)-5,11-dihydrothiéno[3',2':4,5]pyrimido[1,2-a]benzimidazole-7-carboxamide

A une solution de 4-amino-*N,N*-bis(3-méthylbutyl)-3-[(3-pipéridin-1-ylpropyl)amino] benzamide (1,55 g) dans le méthanol (20 ml) sont successivement additionnés le méthyle 3-isothiocyanatothiophene-2-carboxylate (890 mg), l'oxyde de mercure II jaune (1,6 g) et le soufre (30 mg). Le mélange est chauffé à reflux pendant 16 h. Après refroidissement, le mélange est filtré sur célite et le filtrat est concentré sous pression réduite à 40° C. La purification du résidu par chromatographie éclair sur gel de silice (éluant : dichlorométhane 100 % à dichlorométhane/méthanol 95:5) donne le composé attendu sous forme de base libre. Le sel de chlorhydrate correspondant est formé par addition d'une solution HC1 1N dans l'éther éthylique à la solution de la base libre dans l'acétate d'éthyle. Le précipité obtenu est filtré et séché pour donner le composé chlorhydrate attendu (920 mg).

SM/CL : MM calculée = 550,3 ; m/z = 549,8 (MH+)

RMN (¹H, 400 MHz, DMSO-d₆): δ 0,68 (s, 6H), 0,95 (s, 6H), 1,25-1,80 (m, 12H), 2,27 (m, 2H), 2,80 (m, 2H), 3,18 (m, 4H), 3,35 (d, 2H), 3,45 (m, 2H), 4,43 (t, 2H), 7,32 (AB, 1H), 7,35 (AB, 1H), 7,77 (s, 1H), 8,25 (AB, 1H), 8,52 (AB, 1H), 9,72 (s, 1H).

### Exemple A2 : chlorhydrate de N,N bis(3-méthylbutyl)-12-oxo-5-(3-pipéridin-1-ylpropyl)-5,12-dihydro[1]benzotbiéno[3',2':4,5]pyrimido [1,2-a]benzimidazole-3-carboxamide

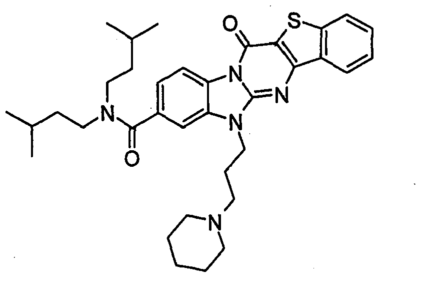

A une suspension de résine *N*-méthylcyclohexylcarbodiimide -*N*-méthyl polystyrène (265 mg, charge de 1,7 mmol/g, 3 eq) dans le tétrahydrofuranne (1 ml), placée dans un tube réactionnel "Personal Chemistry^{®}", sont successivement additionnées une solution de 4-amino-*N*,*N*-bis(3-méthylbutyl)-3-[(3-pipéridin-1-ylpropyl)amino]benzamide (62 mg) dans le tétrahydrofuranne (1 ml) et une solution de méthyle 3-isothiocyanatothiophène-2-carboxylate (45 mg) dans le tétrahydrofuranne (1 ml). Le tube est scellé par une capsule, placé dans le micro-onde "Personal Chemistry^{®}" et chauffé sous agitation magnétique à 100° C pendant 15 minutes. Le mélange est ensuite filtré et le filtrat est concentré sous pression réduite à 40° C. Une solution du résidu dans le méthanol (3 ml) et la triéthylamine (30 mg) est placée dans un tube réactionnel "Personal Chemiste^{®}" et chauffée au micro-onde sous agitation magnétique à 100° C pendant 10 minutes. Le mélange est ensuite concentré sous pression réduite à 40° C. La purification du résidu par chromatographie éclair sur gel de silice (éluant : dichlorométhane 100 % à dichlorométhane/méthanol 90:10) donne le composé attendu sous forme de base libre. Le sel de chlorhydrate correspondant est formé par addition d'une solution HCl 1N dans l'éther éthylique à la solution de la base libre dans l'acétate d'éthyle. Le précipité obtenu est filtré et séché pour donner le composé chlorhydrate attendu (55 mg).

SM/CL : MM calculée = 600,4 ; m/z = 599,8 (MH+)

RMN (¹H, 400 MHz, DMSO-d₆) : δ 0,68 (s, 6H), 0,96 (s, 6H), 1,21-1,81 (m, 12H), 2,20 (m, 4H), 3,20 (m, 2H), 3,30 (m, 2H), 3,45 (m, 2H), 4,54 (t, 2H), 7,37 (AB, 1H), 7,61 (m, 1H), 7,69 (m, 1H), 7,81 (s, 1H), 8,14 (AB, 1H), 8,32 (AB, 1H), 8,58 (AB, 1H), 9,1 (s,1H).

### Préparation d'isothiocyanates non commerciaux :

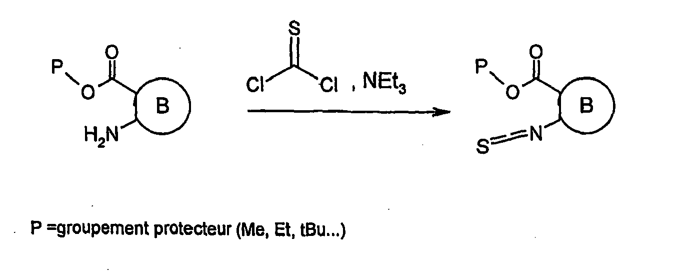

Une amine primaire peut être convertie en isothiocyanate, par traitement avec du thiophosgène en présence d'une base tertiaire telle que la triéthylamine, diisopropyléthylamine, dans un solvant aprotique tel que le dichlorométhane ou le tétrahydrofuranne, à une température de 0-20° C pendant 0,1 à 2 heures.

### Préparation du méthyle 3-isothiocyanatothiophene-2-carboxylate

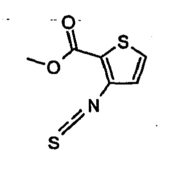

A une solution refroidie à 0° C, de 3-amino-2-thiophènecarboxylate (2 g, 1 eq) et de triéthylamine (3,9 g, 3 eq) dans le tétrahydrofuranne (100 ml) est additionné goutte à goutte le thiophosgène (1,1 ml, 1,1 eq). Le mélange est agité 15 min à 0° C puis additionné d'eau (70 ml) et d'éther diéthylique (150ml). Après décantation et extractions, les phases organiques sont réunies, lavées avec de la saumure, séchées sur Na₂SO₄ puis concentrées sous pression réduite à 40° C. La purification du résidu par chromatographie éclair sur gel de silice (éluant : heptane 100 % à heptane/acétate d'éthyle 7:3) donne le composé attendu sous forme de poudre beige (2,15 g, 85 % rendement).

RMN (¹H, 400 MHz, DMSO-d₆) : δ 3,84 (s, 3H), 7,26 (d, 1H), 7,98 (d, 1H).

Les isothiocyanates suivants ont été préparés selon une procédure analogue à celle décrite pour le méthyle 3-isothiocyanatothiophène-2-carboxylate :

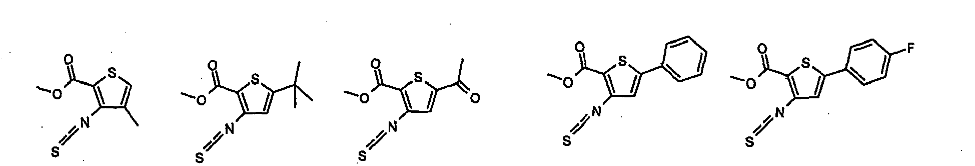

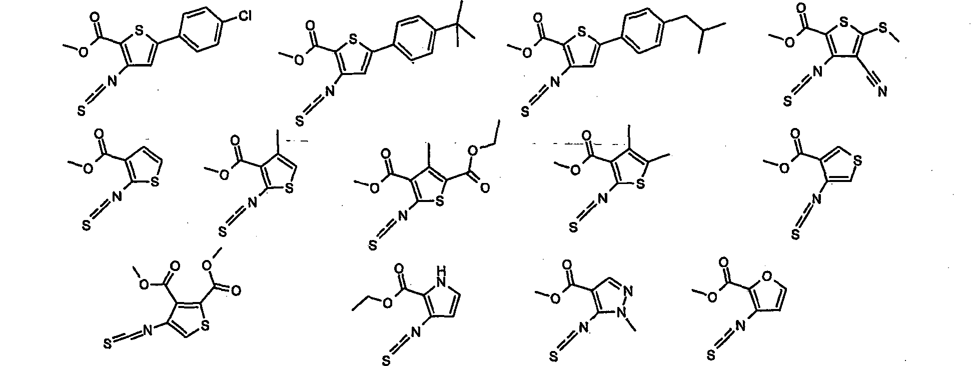

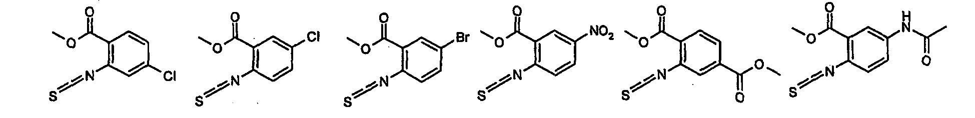

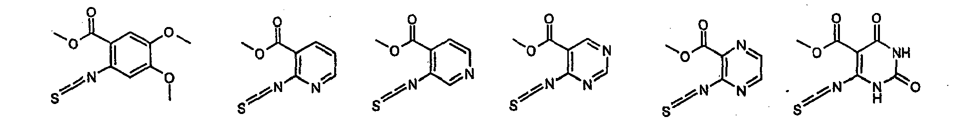

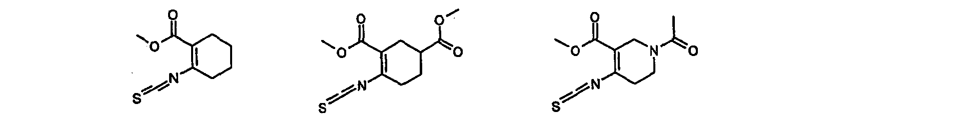

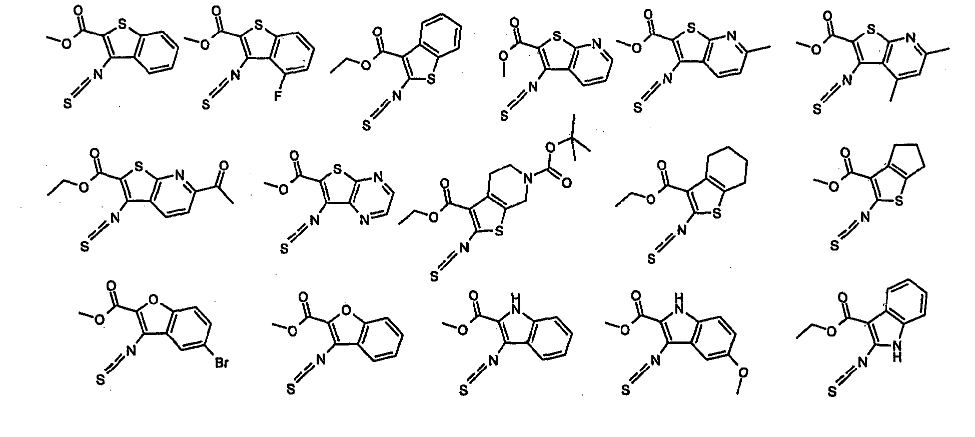

Selon le schéma réactionnel A et de façon analogue à la procédure décrite pour la synthèse du chlorhydrate de *N*,*N*-bis(3-méthylbutyl)-11-oxo-5-(3-pipéridin-1-ylpropyl)-5,11-dihydrothiéno[3',2':4,5]pyrimido[1,2-*a*]benzimidazole-7-carboxamide ou du chlorhydrate de *N,N* bis(3-méthylbutyl)-12-oxo-5-(3-pipéridin-1-ylpropyl)-5,12-dihydro[1]benzothiéno [3',2':4,5]pyrimido[1,2-*a*]benzimidazole-3-carboxamide, les composés suivants ont été préparés : 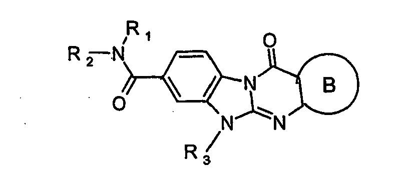 dans lesquels R₁R₂N représente l'un des radicaux ci-après : 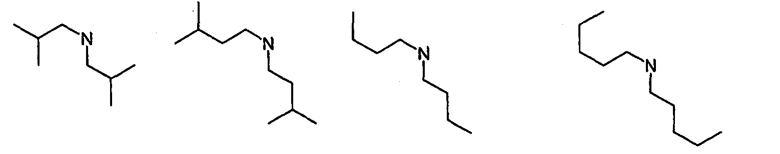 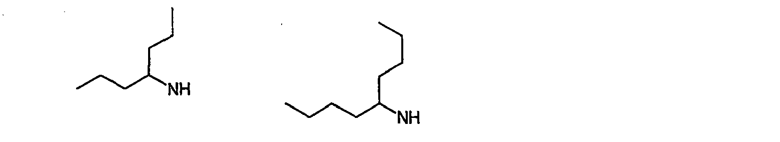 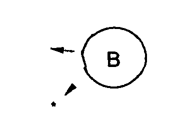 représente l'un des radicaux ci-après : 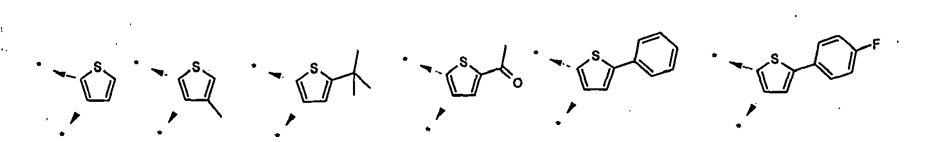 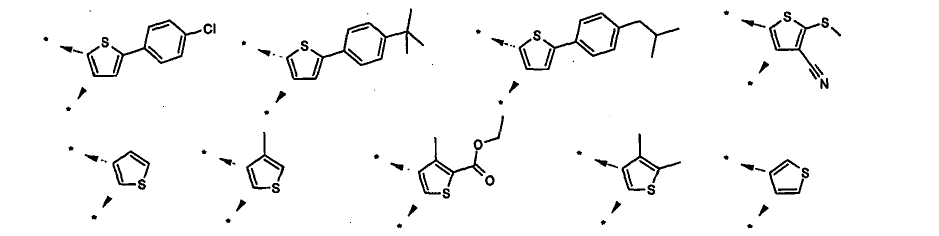 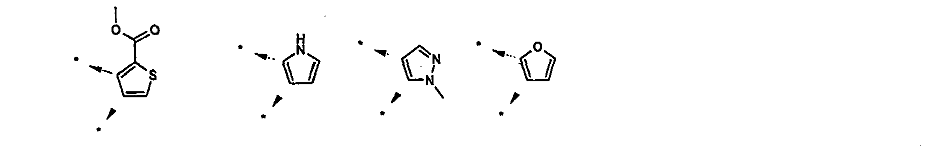 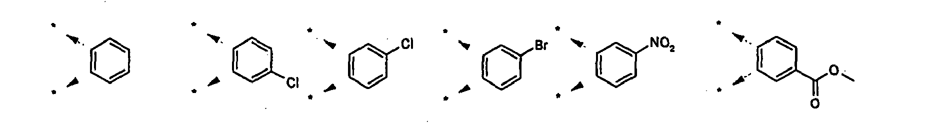 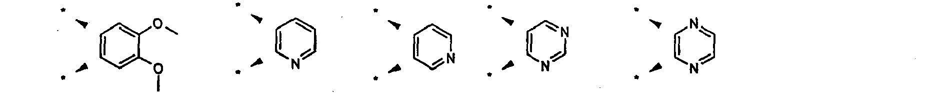 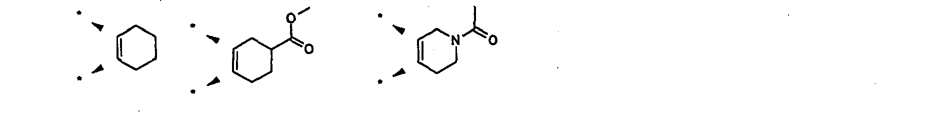 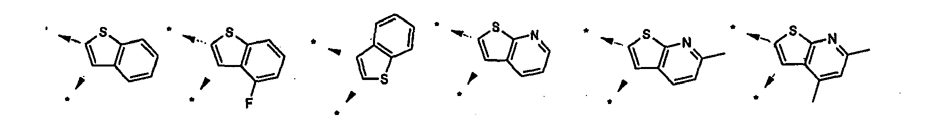 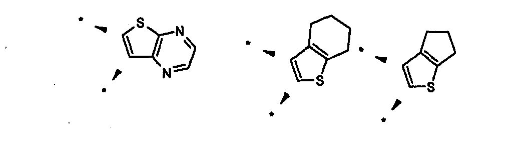 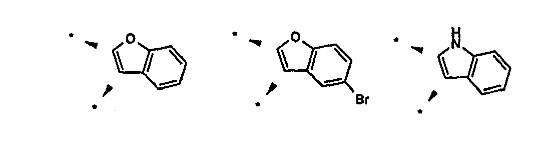 et R₃ représente l'un des radicaux ci-après :

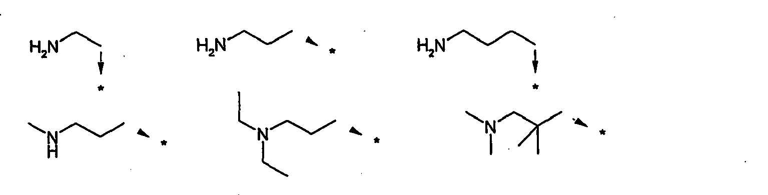

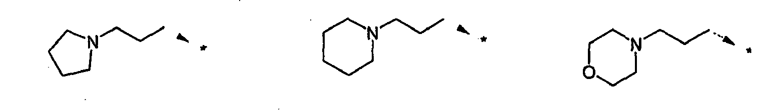

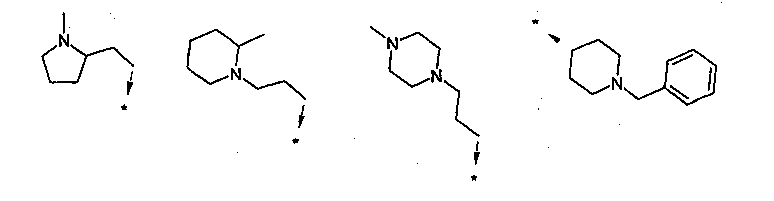

### B. Préparation selon le schéma réactionnel B :

Les composés de formule (I) selon l'invention dans laquelle A représente -C(O)-, peuvent être également préparés selon le schéma B suivant :

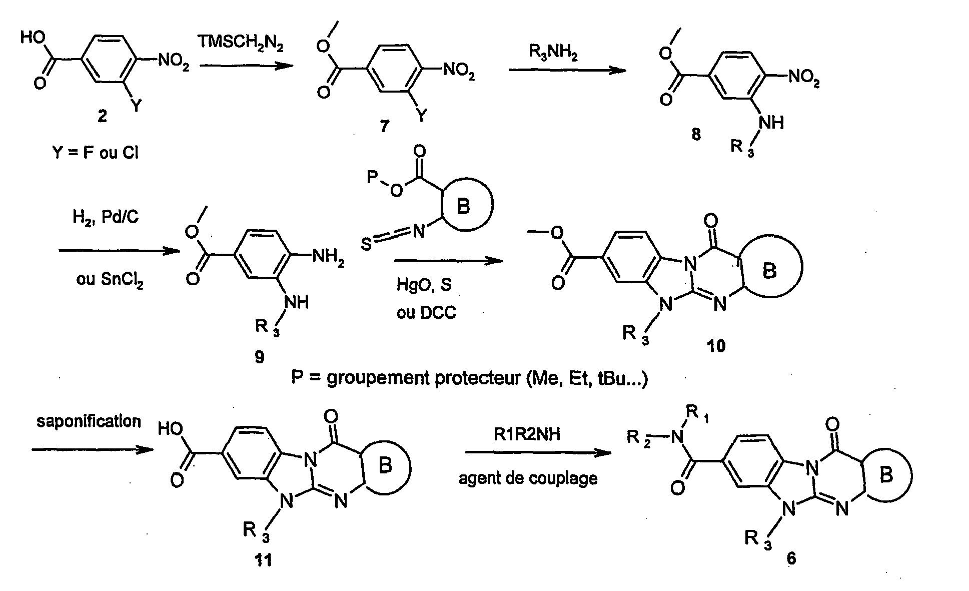

Comme décrit dans le schéma B, l'acide carboxylique (2) peut être converti en ester méthylique (7) soit par traitement avec une solution de triméthylsilyl-diazométhane à une température de 0-20° C, soit par formation d'un sel de carboxylate à l'aide d'une base inorganique telle que l'hydroxyde de lithium dihydrate ou le carbonate de césium, à température ambiante pendant 30 min à 2 heures, dans un solvant organique inerte tel que le tétrahydrofuranne, suivi de l'addition de diméthylsulfate à température ambiante et agitation à reflux pendant 5 à 15 heures. Le dérivé fluoré ou chloré (7) peut-être traité par une amine primaire en présence d'une base inorganique telle que le carbonate de césium ou de potassium dans un solvant organique inerte tel que le diméthylformamide ou facétonitrile à une température de 20-100° C pendant 2 à 48 heures pour conduire au dérivé (8). La fonction nitro du composé (8) peut-être réduite par traitement avec du chlorure d'étain dihydrate dans un solvant inerte telle que l'acétate d'éthyle ou le diméthylformamide, à une température de 60-80° C pendant 3 à 15 heures, ou par hydrogénation catalytique en présence de palladium sur charbon 10 % dans un solvant inerte tel que le méthanol, l'éthanol, l'acétate d'éthyle ou un mélange de ces solvants, à une température de 18-25° C, pendant 2 à 8 heures, pour conduire à la dianiline (9). Le dérivé (9) est ensuite ensuite traité par un isothiocyanate ortho-ester en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène, en présence ou non d'une base organique telle que la triéthylamine ou diisopropyléthylamine, dans un solvant inerte tel que le tétrahydrofuranne, chlorure de méthylène, ou chloroforme à une température de 20-70° C pendant 2 à 72 heures, ou sous micro-ondes (équipement Personal Chemistry^{®}), dans un tube fermé, à 100° C pendant 10 à 30 minutes pour conduire au dérivé (10). Alternativement, le dérivé (9) peut être traité par un isothiocyanate ortho-ester en présence d'oxyde de mercure (II) jaune et d'une quantité catalytique de soufre, en présence ou non d'une base organique telle que la triéthylamine, diisopropyléthylamine, méthylate, éthylate ou *tert*-butylate de sodium, dans un solvant polaire tel que le méthanol ou l'éthanol pendant 2 à 24 heures à une température de 20-80° C, ou sous micro-ondes (équipement Personal Chemistry^{®}), dans un tube scellé, à 100° C pendant 10 à 30 minutes pour conduire à (10). L'ester méthylique (10) peut ensuite être saponifié en présence d'une base inorganique telle que l'hydroxyde de lithium dihydrate dans un mélange de solvants polaires tels que l'eau et le tétrahydrofuranne à une température de 20 à 70° C pendant 3 à 17 heures. L'acide carboxylique résultant (11) peut être couplé avec une amine primaire ou secondaire en présence d'un agent de couplage tel que le diisopropylcarbodiimide (DIC), le dicyclohexylcarbodiimide (DCC), le chlorhydrate de 1-(3-dimethylaminopropyl)-3-éthylcarbodiimide (EDC) ou le carbonyldiimidazole (CDI), avec ou sans du 1-hydroxybenzotriazole (HOBt) dans un solvant organique inerte tel que du chlorure de méthylène, tétrahydrofuranne ou diméthylformamide à température ambiante pendant 3 à 24 heures pour conduire à l'amide correspondante (6).

### C. Préparation selon le schéma réactionnel C :

Les composés de formule (I) selon l'invention dans laquelle A représente -CH₂-, peuvent être préparés selon les schémas C et C' suivants :

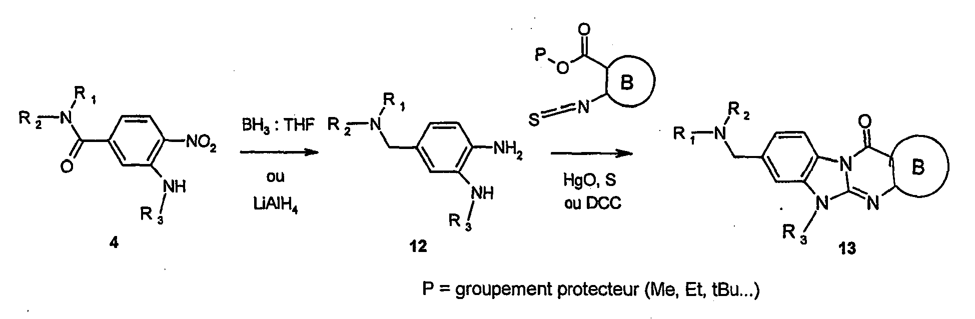

Comme décrit dans le schéma C, le dérivé (4) préparé selon le schéma réactionnel A, peut être réduit en composé (12) à l'aide de borane ou d'hydrure de lithium aluminium dans un solvant aprotique tel que le tétrahydrofuranne ou l'éther diéthylique à une température de 0 à 70° C, pendant 18 à 24 heures. La dianiline (12) peut être ensuite traitée par un isothiocyanate ortho-ester en présence d'un agent de couplage supporté sur une résine ou non tel que le diisopropylcarbodiimide ou dicyclohexylcarbodiimide ou la résine *N*-méthylcyclohexylcarbodiimide *N*-méthyl polystyrène, en présence ou non d'une base organique telle que la triéthylamine ou diisopropyléthylamine, dans un solvant inerte tel que le tétrahydrofuranne, chlorure de méthylène, ou chloroforme à une température de 20-70° C pendant 2 à 72 heures, ou sous micro-ondes (équipement Personal Chemistry^{®}), dans un tube fermé, à 100° C pendant 10 à 30 minutes pour conduire au dérivé (13). Alternativement, le dérivé (12) peut être traité par un isothiocyanate ortho-ester en présence d'oxyde de mercure (II) jaune et d'une quantité catalytique de soufre, en présence ou non d'une base organique telle que la triéthylamine, diisopropyléthylamine, méthylate, éthylate ou *tert-*butylate de sodium, dans un solvant polaire tel que le méthanol ou l'éthanol pendant 2 à 24 heures à une température de 20-80° C, ou sous micro-ondes (équipement Personal Chemistry^{®}), dans un tube scellé, à 100° C pendant 10 à 30 minutes pour conduire à (13).

### Préparation selon le schéma réactionnel C' :

Les composés (13) peuvent également être préparés selon le schéma C' suivant :

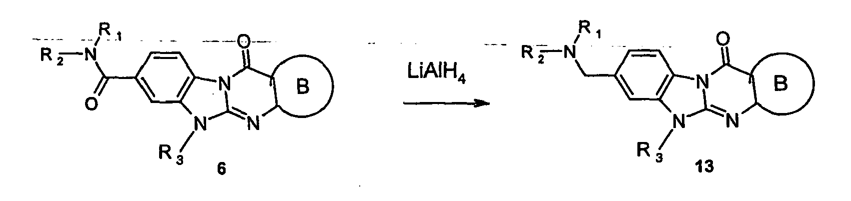

Comme décrit dans le schéma C', l'amide (6) préparée selon les schémas réactionnels A ou B, peut être réduite en amine correspondante (13) à l'aide de borane ou d'hydrure de lithium aluminium dans un solvant aprotique tel que le tétrahydrofuranne ou l'éther diéthylique à une température de 0 à 70° C, pendant 1 à 6 heures.

### Exemple C1 : dichlorhydrate de 8-{[bis(3-méthylbutyl)amino]méthyl}-10-(3-piperidin-1-ylpropyl)thiéno[2',3':4,5]pyrimido [1,2-a]benzimidazol-4(10H)-one

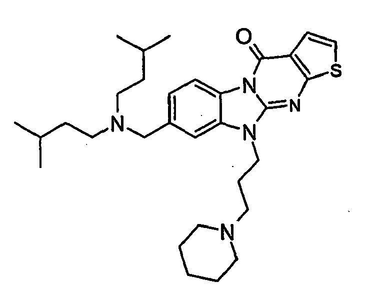

### Etape 1: 4-{[bis(3-méthylbutyl)amino]méthyl}-N²-(3-pipéridin-1-ylpropyl)benzène-1,2-diamine

A une solution refroidie à 0° C de *N*,*N*-bis(3-méthylbutyl)-4-nitro-3-[(3-pipéridin-1-ylpropyl)amino]benzamide (1,6 g) est additionnée goutte à goutte une solution d'hydrure de lithium aluminium (36 ml ; 1N dans le THF). Le mélange est ramené à une température de 20° C puis chauffé au reflux pendant 6 heures et hydrolysé par de l'eau refroidie à 0° C suivie d'une solution de soude 1N. Après addition de dichlorométhane, le mélange est filtré sur célite. Après décantation du filtrat et extractions, les phases organiques réunies sont lavées à la soude 1N puis à la saumure, séchées sur Na₂SO₄ et concentrées sous pression réduite à 40° C pour donner le composé attendu sous forme d'une huile (1,23 g, 85 % rendement).

SM/CL : MM calculée = 402,7 ; m/z = 403,3 (MH+)

RMN (¹H, 400 MHz, DMSO*-d₆*) : δ0,81 (d, 12H), 1,28 (m; 4H), 1,38-(m, 2H), 1,48 (m, 6H), 1,71 (m, 2H), 2,31 (m, 10H), 3,01 (m, 2H), 3,29 (m, 2H), 4,28 (m, 2H), 4,6 (m, 1H), 6,30 (AB, 1H), 6,38 (s, 1H), 6,43 (AB, 1H).

### Etape 2 : Dichlorhydrate de 8-{[bis(3-méthylbutyl)amino]méthyl}-10-(3-pipéridin-1-ylpropyl)thiéno[2',3':4,5]pyrimido[1,2-a]benzimidazol-4(10H)-one

A une solution de 4-amino-*N,N*-bis(3-méthylbutyl)-3-[(3-pipéridin-1-ylpropyl)amino] benzamide (60 mg) dans le méthanol (3 ml) sont successivement additionnés le méthyle 2-isothiocyanatothiophene-3-carboxylate (45 mg), l'oxyde de mercure II jaune (65 mg) et le soufre (3 mg). Le mélange est chauffé à reflux pendant 16 h. Après refroidissement, le mélange est filtré sur célite et le filtrat est concentré sous pression réduite à 40° C. La purification du résidu par chromatographie éclair sur gel de silice (éluant : dichlorométhane 100 % à dichlorométhane/méthanol 90:10) donne le composé attendu sous forme de base libre. Le sel de chlorhydrate correspondant est formé par addition d'une solution HCl 1N dans l'éther éthylique à la solution de la base libre dans l'acétate d'éthyle. Le précipité obtenu est filtré et séché pour donner le composé chlorhydrate attendu (30 mg).

SM/CL : MM calculée = 536,4 ; m/z = 535,8 (MH+)

RMN (¹H, 400 MHz, DMSO-d₆) : δ0,86 (d, 12H), 1,30 (m, 2H), 1,54 (m, 2H), 1,68 (m, 8H), 2,39 (m, 2H), 2,78 (m, 2H), 3,01 (m, 4H), 3,19 (m, 2H), 3,38 (m, 2H), 4,37 (t, 2H), 4,48 (m, 2H), 7,37 (AB, 1H), 7,54 (AB, 1H), 8,26 (AB, 1H), 8,30 (s, 1H), 8,51 (AB, 1H), 9,97 (s, 1H), 10,96 (s, 1H).

Les composés suivants ont été préparés selon les schémas réactionnels C ou C': 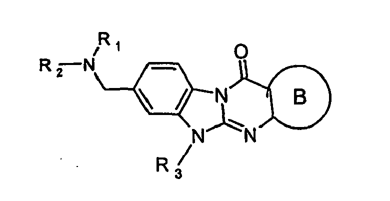 dans lesquels
R₁R₂N représente l'un des radicaux ci-après : 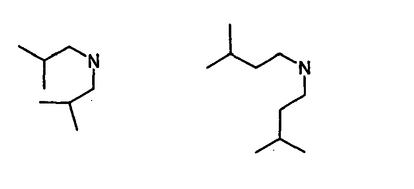 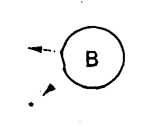 représente l'un des radicaux ci-après : 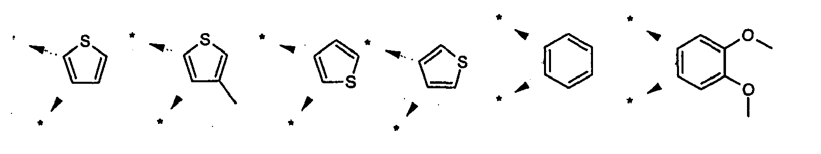
et R₃ représente l'un des radicaux ci-après :

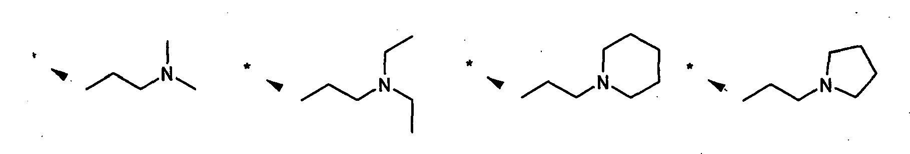

L'invention a également pour objet un procédé de préparation d'un composé de formule (I) tel que défini ci-dessus, caractérisé en ce que l'on traite le composé de formule générale : 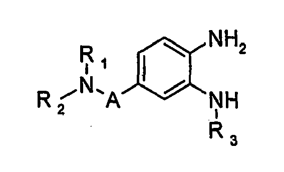 dans laquelle A, R₁, R₂, R₃ ont la signification indiquée ci-dessus, par un isothiocyanate ortho-ester de formule générale 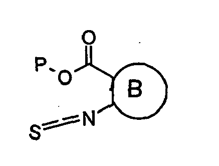 dans laquelle P est un groupement protecteur tel que méthyle, éthyle, *tert*-butyl et B a la signification indiquée ci-dessus en présence d'un agent de couplage ou d'oxyde de mercure (II) jaune en présence de soufre, dans un solvant protique ou aprotique, en présence ou non d'une base. La réaction peut être réalisée à une température de 50 à 80° C pendant une durée de 3 à 48 heures. La réaction peut également être réalisée sous micro-ondes, dans un tube scellé, à une température de 80-120° C, pendant 5 à 30 minutes.

L'agent de couplage peut être supporté tel que la résine *N*-méthylcyclohexylcarbodiimide *N*-méthyl polystyrène ou non supporté tel que diisopropylcarbodiimide, diéthylcarbodiimide ou dicyclohexylcarbodiimide. On peut utiliser un solvant protique tel que le méthanol ou l'éthanol ou aprotique tel que le tétrahydrofuranne ou l'acétonitrile. La base peut-être la triéthylamine, la diisopropyléthylamine, le méthylate, éthylate ou *tert*-butylate de sodium.

Les composés 1 de la présente invention possèdent d'intéressantes propriétés pharmacologiques. C'est ainsi que l'on a découvert que les composés I de la présente invention possèdent une bonne affinité pour certains sous-types de récepteurs des mélanocortines, en particulier des récepteurs MC4.

Les composés de la présente invention peuvent ainsi être utilisés dans différentes applications thérapeutiques. Ils peuvent avantageusement être utilisés pour traiter les états pathologiques ou les maladies dans lesquels un ou plusieurs récepteurs des mélanocortines sont impliqués tels que les états inflammatoires, les désordres pondéraux (l'obésité, la cachexie, l'anorexie), les désordres de l'activité sexuelle (les troubles de l'érection), la douleur, mais également les troubles mentaux (l'anxiété, la dépression), les toxicomanies, les maladies de la peau (l'acné, les dermatoses, les mélanomes). On trouvera ci-après, dans la partie expérimentale, une illustration des propriétés pharmacologiques des composés de l'invention.

La présente demande a également pour objet des compositions pharmaceutiques contenant, à titre de principe actif, au moins un produit de formule 1 telle que définie ci-dessus, ainsi que les sels pharmaceutiquement acceptables dudit produit de formule I, en association avec un support pharmaceutiquement acceptable.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

La présente demande a également pour objet l'utilisation des composés selon la présente invention, pour la préparation d'un médicament pour le traitement des désordres pondéraux tels que l'obésité, la cachexie et plus particulièrement la cachexie des pathologies cancéreuses, cachexie du sida, cachexie de la personne âgée, cachexie cardiaque, cachexie rénale, cachexie de l'arthrite rhumatoïde, et l'anorexie, le traitement de la douleur et plus particulièrement la douleur neuropathique, des troubles mentaux tels que l'anxiété et la dépression, des désordres de l'activité sexuelle tels que les troubles de l'érection.

La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale :

Les composés selon l'invention obtenus selon les procédures des exemples A, B, C et C' précédemment décrites, sont rassemblés dans le tableau ci-dessous.

Les composés sont caractérisés par leur temps de rétention (tr) et leur pic moléculaire déterminé par spectrométrie de masse (MH+).

Pour la spectrométrie de masse, un spectromètre de masse simple quadripôle (Micromass, modèle Platform) equipé d'une source *electrospray* est utilisé avec une résolution de 0,8 Da à 50 % de vallée. Un calibrage est effectué mensuellement entre les masses 80 et 1000 Da à l'aide d'un mélange calibrant d'iodure de sodium et d'iodure de rubidium en solution dans un mélange isopropanol/eau (1/1 Vol.).

Pour la chromatographie liquide, un système Waters incluant un dégazeur en ligne, une pompe quaternaire Waters 600, un injecteur plaque Gilson 233 et un détecteur UV Waters PAD 996, est utilisé.

Les conditions d'élution employées sont les suivantes:
Eluant : **A** eau + 0,04 % acide trifluoroacétique ; **B** acétonitrile

| **T (min)** | **A%** | **B%** |
|---|---|---|
| 1 | 95 | 5 |
| 8,5 | 5 | 95 |
| 10,5 | 5 | 95 |
| 10,6 | 95 | 5 |
| 14,9 | 95 | 5 |
| 15,0 | 95 | 5 |

Débit : 1 ml/min ; Injection : 10 µl ; Colonne : Uptisphere ODS 3 µm 75*4,6 mm i.d.

Ces exemples sont présentés pour illustrer les procédures ci-dessus et ne doivent en aucun cas être considérés comme une limite à la portée de l'invention.

| **Exemples** | **Structures moléculaires** | **[M+H]+** | **tr (min)** |
|---|---|---|---|
| 1 | 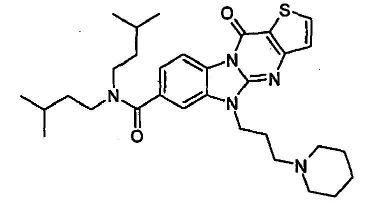 | 550,3 | 8,9 |
| 2 | 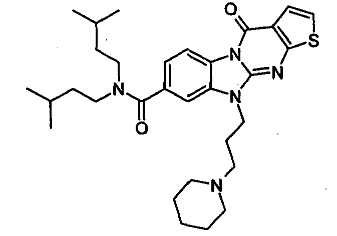 | 550,4 | 9,6 |
| 3 | 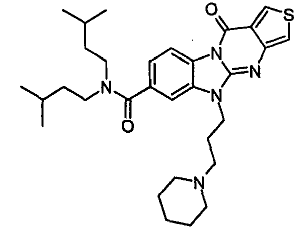 | 550,4 | 9,5 |
| 4 | 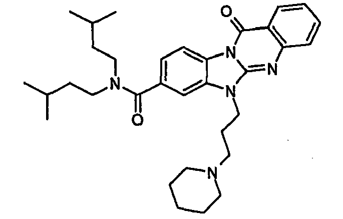 | 544,1 | 9,1 |
| 5 | 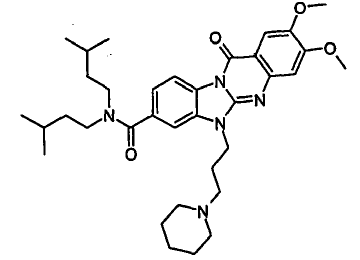 | 604,4 | 9,6 |
| 6 | 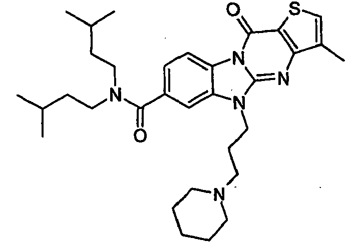 | 564,4 | 9,9 |
| 7 | 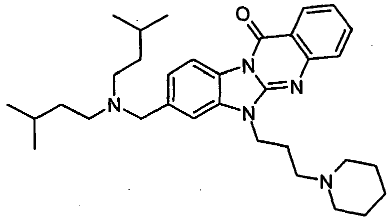 | 530,5 | 8,1 |
| 8 | 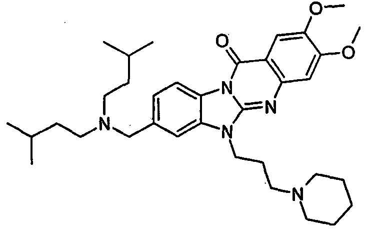 | 590,4 | 8,1 |
| 9 | 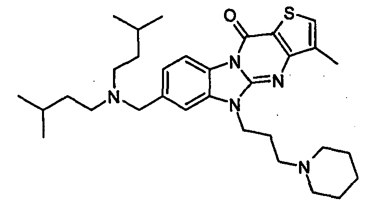 | 550,4 | 8,2 |
| 10 | 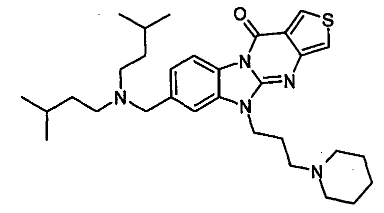 | 536,4 | 8,0 |
| 11 | 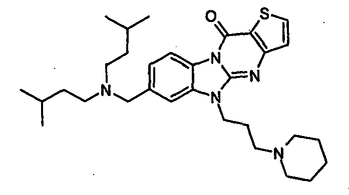 | 536,4 | 8,0 |
| 12 | 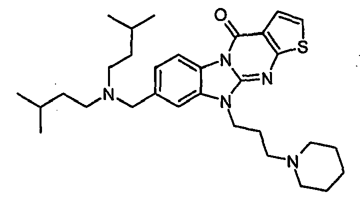 | 536,4 | 8,0 |
| 13 | 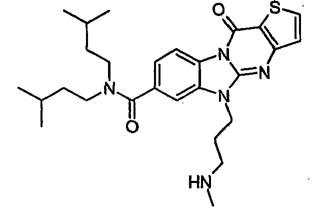 | 496,5 | 9,2 |
| 14 | 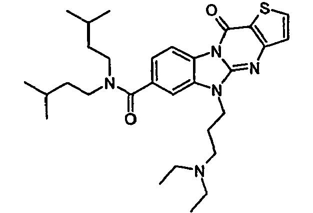 | 538,5 | 9,5 |
| 15 | 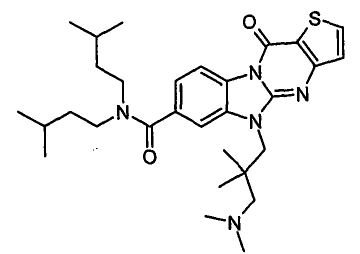 | 538,5 | 9,5 |
| 16 | 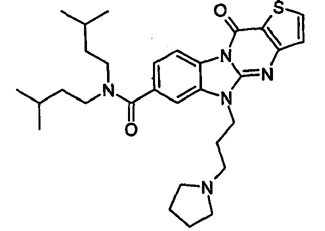 | 536,3 | 9,2 |
| 17 | 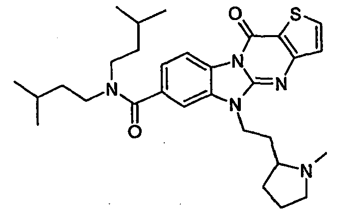 | 536,5 | 9,4 |
| 18 | 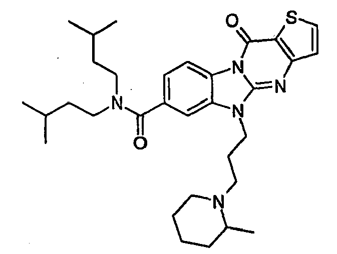 | 564,5 | 9,6 |
| 19 | 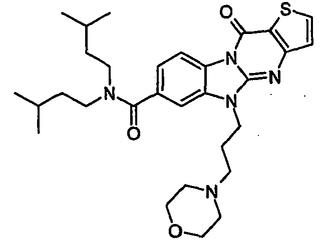 | 552,5 | 9,3 |
| 20 | 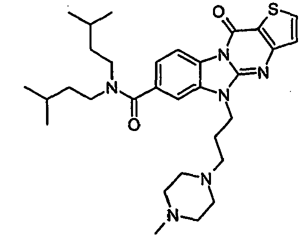 | 565,5 | 9,0 |
| 21 | 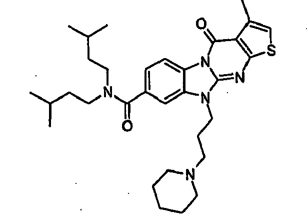 | 564,2 | 10,1 |
| 22 | 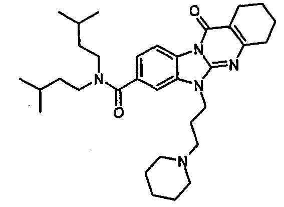 | 548,4 | 9,6 |
| 23 | 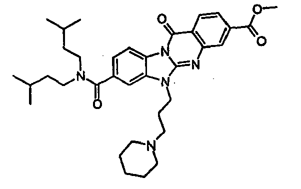 | 602,3 | 10,0 |
| 24 | 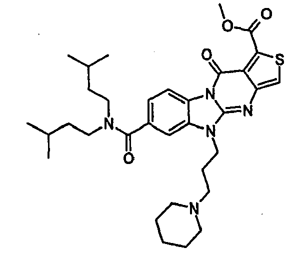 | 608,4 | 9,5 |
| 25 | 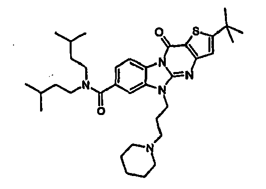 | 606,4 | 10,4 |
| 26 | 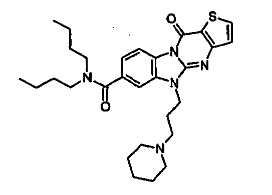 | 522,3 | 9,1 |
| 27 | 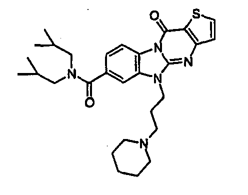 | 522,3 | 9,0 |
| 28 | 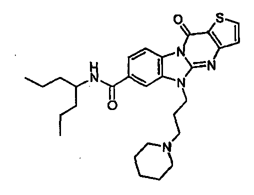 | 508,3 | 8,9 |
| 29 | 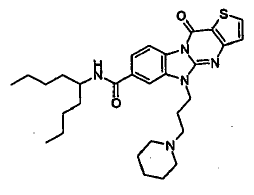 | 536,3 | 9,4 |
| 30 | 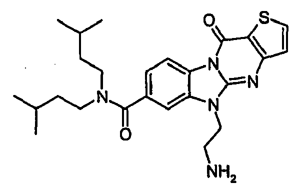 | 468,4 | 9,1 |
| 31 | 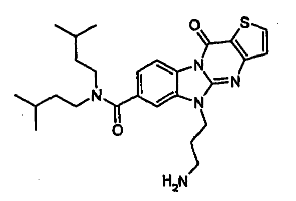 | 482,4 | 9,1 |
| 32 | 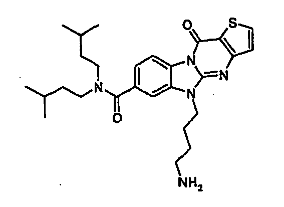 | 496,4 | 9,1 |
| 33 | 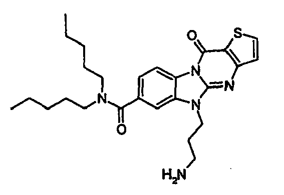 | 482,4 | 9,3 |
| 34 | 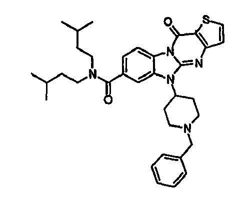 | 598,3 | 10,0 |
| 35 | 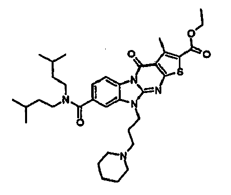 | 636,5 | 11,1 |
| 36 | 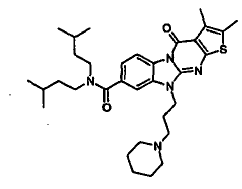 | 578,4 | 10,7 |
| 37 | 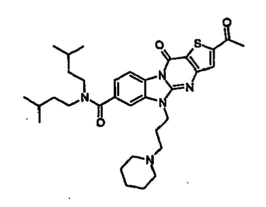 | 592,4 | 9,9 |
| 38 | 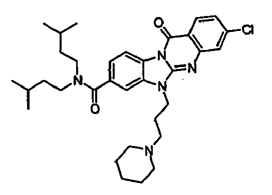 | 578,2 | 10,1 |
| 39 | 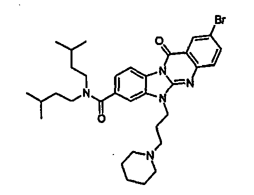 | 622,2 | 10,3 |
| 40 | 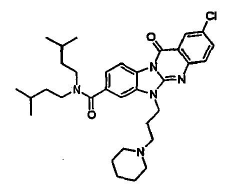 | 578,2 | 10,2 |
| 41 | 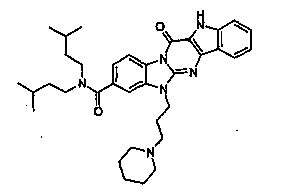 | 583,4 | 9,3 |
| 42 | 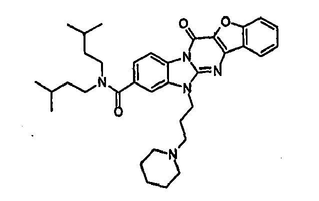 | 584,4 | 9,6 |
| 43 | 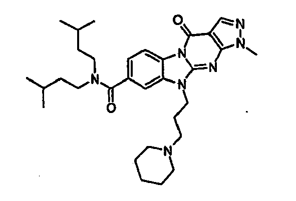 | 548,3 | 9,0 |
| 44 | 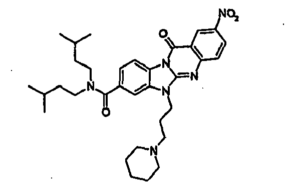 | 589,4 | 9,8 |
| 45 | 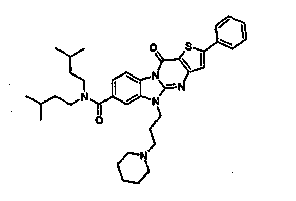 | 626,4 | 10,3 |
| 46 | 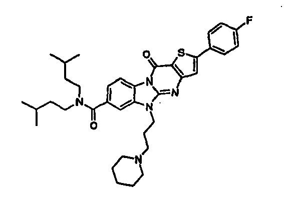 | 644,4 | 10,4 |
| 47 | 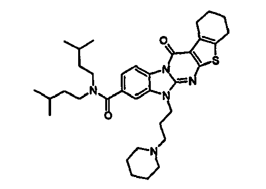 | 604,3 | 10,4 |
| 48 | 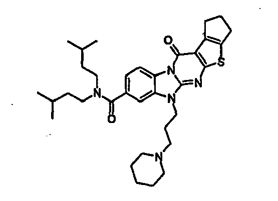 | 590,3 | 10,0 |
| 49 | 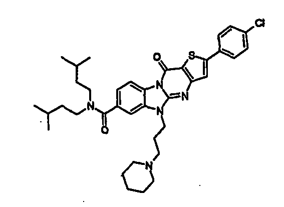 | 660,4 | 10,7 |
| 50 | 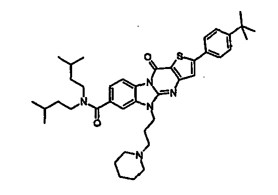 | 682,5 | 11,2 |
| 51 | 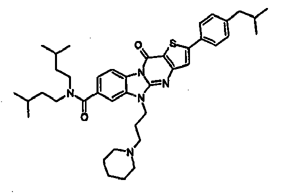 | 682,5 | 11,3 |
| 52 | 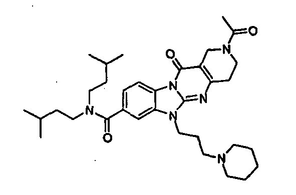 | 591,4 | 8,5 |
| 53 | 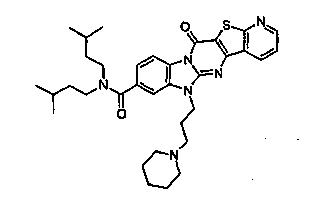 | 601,3 | 9,5 |
| 54 | 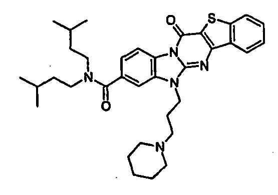 | 600,4 | 10,1 |
| 55 | 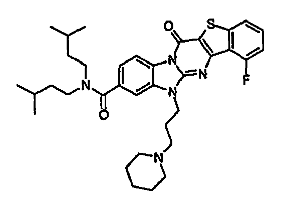 | 618,4 | 10,1 |
| 56 | 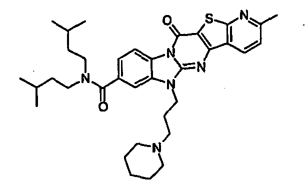 | 615,4 | 9,6 |
| 57 | 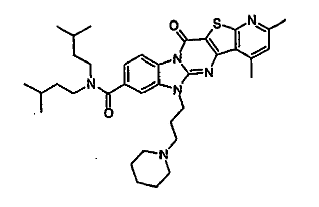 | 629,4 | 9,9 |
| 58 | 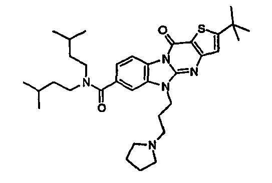 | 592,4 | 10,1 |
| 59 | 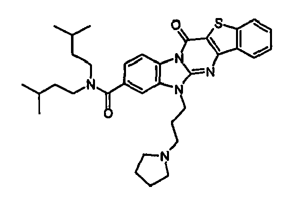 | 586,3 | 10,0 |
| 60 | 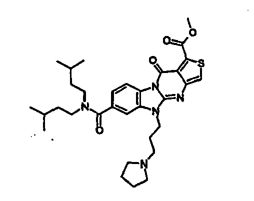 | 594,3 | 9,3 |
| 61 | 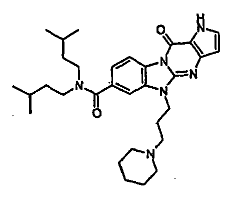 | 533,3 | 9,0 |
| 62 | 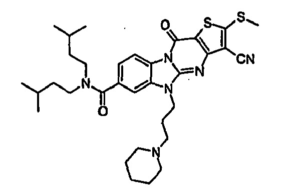 | 621,1 | 9,9 |
| 63 | 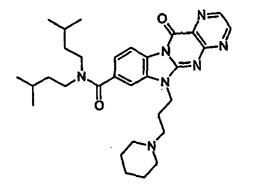 | 546,3 | 8,8 |
| 64 | 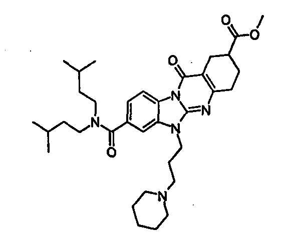 | 606,4 | 9,4 |
| 65 | 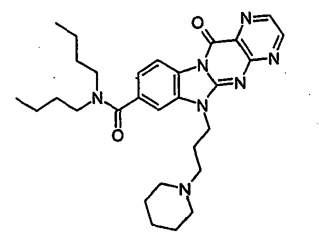 | 518,3 | 8,4 |
| 66 | 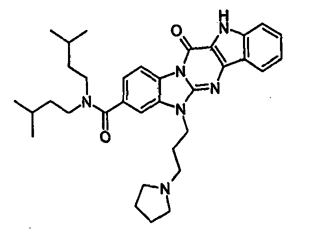 | 569,4 | 9,4 |
| 67 | 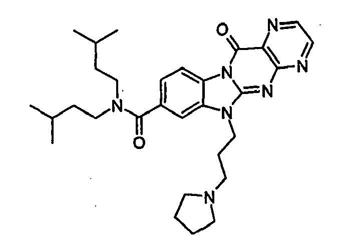 | 532,4 | 8,9 |
| 68 | 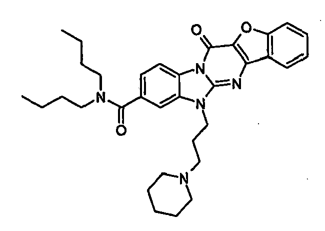 | 556,4 | 9,6 |
| 69 | 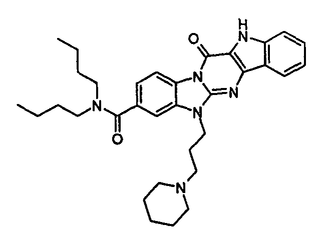 | 554,4 | 9,1 |
| 70 | 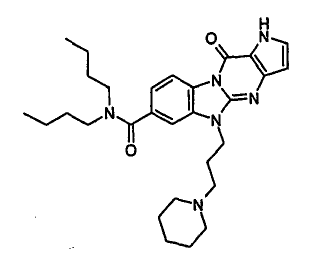 | 505,4 | 8,5 |
| 71 | 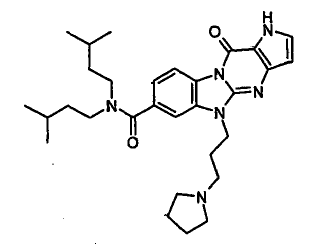 | 519,4 | 8,9 |
| 72 | 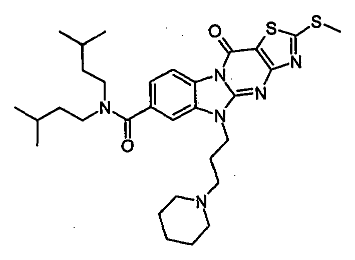 | 597,4 | 9,9 |
| 73 | 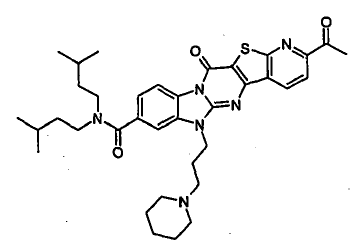 | 643,4 | 10,3 |
| 74 | 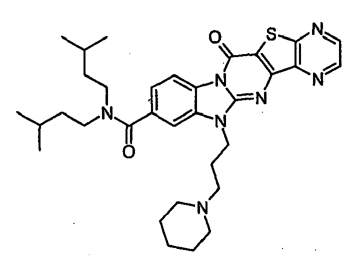 | 602,4 | 9,6 |
| 75 | 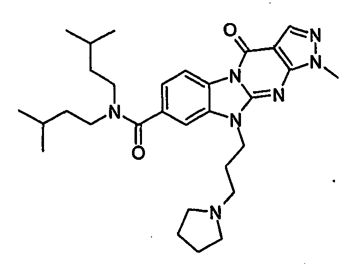 | 534,4 | 9,1 |
| 76 | 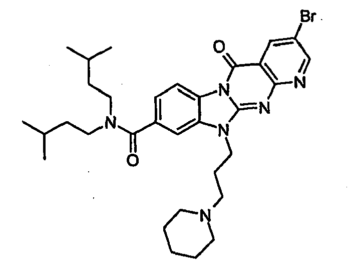 | 623,3 | 9,7 |
| 77 | 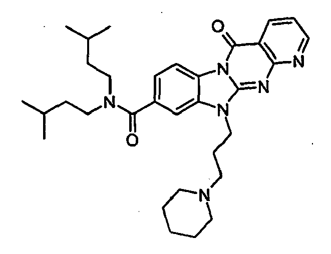 | 545,4 | 9,0 |
| 78 | 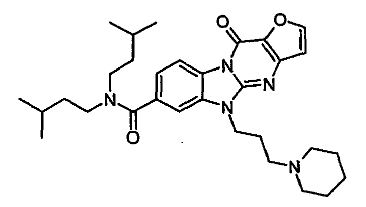 | 534,3 | 9,1 |

### Etude pharmacologique

L'affinité des composés de la présente invention pour les différents sous-types de récepteurs des mélanocortines a été mesurée selon les procédures analogues à celles décrites ci-après pour les récepteurs MC4.

### Etude de l'affinité des composés pour les récepteurs MC4 des mélanocortines :

L'affinité des composés de l'invention pour les récepteurs MC4 est déterminée par la mesure de l'inhibition de la liaison de la [¹²⁵I]-[Nle⁴ D-Phe⁷]-α-MSH à des préparations membranaires de cellules CHO-K1 transfectées.

Les cellules CHO-K1 exprimant de façon stable les récepteurs MC4 humains sont cultivées dans un milieu RPMI 1640 contenant 10 % de sérum foetal de veau, 2 mM de glutamine, 100 U/ml de pénicilline, 0,1 mg/ml de streptomycine et 0,5 mg/ml de G418. Les cellules sont collectées avec 0,5 mM d'EDTA et centrifugées à 500 g pendant 5 min à 4° C. Le culot est re-suspendu dans un milieu salin avec tampon phosphate (PBS) et centrifugé à 500 g pendant 5 min à 4° C. Le culot est re-suspendu dans un milieu tampon Tris 50 mM à pH 7,4 et centrifugé à 500 g pendant 5 min à 4° C. Les cellules sont lysées par sonication et centrifugées à 39 000 g pendant 10 min à 4° C. Le culot est re-suspendu dans le milieu tampon Tris 50 mM à pH 7,4 et centrifugé à 50 000 g pendant 10 min à 4° C. Les membranes obtenues dans ce dernier culot sont stockées à -80° C.

La mesure de l'inhibition compétitive de la liaison de la [¹²⁵I]-[Nle⁴, D-Phe⁷]-α-MSH sur les récepteurs MC4 est effectuée en duplicats à l'aide de plaques en polypropylène de 96 puits. Les membranes cellulaires (50 µg de protéines/puits) sont incubées avec la [¹²⁵I]-[Nle⁴, D-Phe⁷]-α-MSH (0,5 nM) pendant 90 min à 37° C dans un milieu tampon Tris-HCl 50 mM, pH 7,4, comprenant 0,2 % d'albumine bovine de sérum (BSA), 5 mM de MgCl₂, et 0,1 mg/ml de bacitracine.

La [¹²⁵I]-[Nle⁴, D-Phe⁷]-α-MSH liée est séparée de la [¹²⁵I]-[Nle⁴, D-Phe⁷]-α-MSH libre par filtration à travers des plaques de filtres en fibre de verre GF/C (Unifilter, Packard) pré-imprégné avec 0,1 % de polyéthylènimine (P.E.I.), en utilisant un Filtermate 196 (Packard). Les filtres sont lavés avec du tampon Tris-HCl 50 mM, pH 7,4 à 0-4° C et la radioactivité présente est déterminée à l'aide d'un compteur (Packard Top Count).

La liaison spécifique est obtenue en soustrayant la liaison non spécifique (déterminée en présence de 0,1 µM de Nle⁴, D-Phe⁷-α-MSH) de la liaison totale. Les données sont analysées par régression non-linéaire assistée par ordinateur (MDL) et les valeurs des constantes d'inhibition (Ki) sont déterminées.

L'activité agoniste ou antagoniste des récepteurs MC4 des composés de la présente invention a été déterminée en mesurant la production d'AMP cyclique par les cellules CHO-K1 transfectées par le récepteur MC4.

### Mesure de la production d'AMP cyclique intracellulaire via les récepteurs MC4 :

Les cellules CHO-K1 exprimant les récepteurs MC4 des mélanocortines sont cultivées dans des plaques à 384 puits dans un milieu RPMI 1640 avec 10 % de sérum foetal de veau et 0,5 mg/ml de G418. Les cellules sont lavées 2 fois avec 50 µl de milieu RPMI comprenant 0,2 % BSA et 0,5 mM de 3-isobutyl-1-méthylxanthine (IBMX).

Pour mesurer l'effet agoniste d'un composé, les cellules sont incubées pendant 5 min à 37° C en présence de 0,5 mM d'IBMX, puis la stimulation de la production d'AMP cyclique est obtenue en ajoutant le composé à des concentrations comprises entre 1 pM et 10 µM en duplicats pendant 20 min à 37° C. L'effet antagoniste d'un composé est mesuré en inhibant la stimulation de la production d'AMP cyclique induite par la Nle⁴, D-Phe⁷-α-MSH, à des concentrations comprises entre 1 pM et 10 µM, en présence du composé à tester, à des concentrations comprises entre 1 nM et 10 µM, en duplicats pendant 20 min à 37° C.

Le milieu réactionnel est éliminé et 80 µl de tampon de lyse sont ajoutés. Le taux d'AMP cyclique intracellulaire est mesuré par un test de compétition avec de l'AMP cyclique fluorescent (CatchPoint, Molecular Devices).

Les tests effectués selon les protocoles décrits ci-dessus ont permis de montrer que les produits selon la présente invention ont une bonne affinité sur les récepteurs MC4, la constante d'inhibition Kᵢ sur ces récepteurs étant inférieure au micromolaire pour la majorité des composés exemplifiés.

## Revendications

1. Composés de formule générale **(I)** 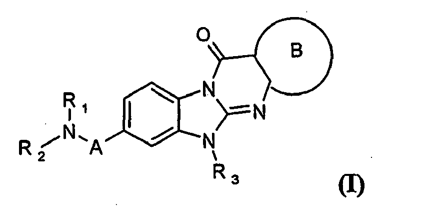 sous forme racémique, d'énantiomère ou toutes combinaisons de ces formes et dans laquelle:
A représente -CH₂- ou -C(O)- ;
R₁ représente l'atome d'hydrogène; un radical (C₁-C₈)alkyle éventuellement substitué par hydroxy ou un ou plusieurs radicaux halo identiques ou différents ; (C₂-C₆)alkényle ; ou un radical de formule -(CH₂)ₙ-X₁ ;
R₂ représente un radical (C₁-C₈)alkyle éventuellement substitué par hydroxy ou un ou plusieurs radicaux halo identiques ou différents ; (C₂-C₆)alkényle ; ou un radical de formule -(CH₂)ₙ-X₁ ;
chaque X₁ représente, indépendamment, un radical (C₁-C₆)alkoxy, (C₃-C₇)cycloalkyle, adamantyle, hétérocycloalkyle, aryle ou hétéroaryle,
les radicaux (C₃-C₇)cycloalkyle, hétérocycloalkyle, aryle et hétéroaryle étant éventuellement substitués par un ou plusieurs substituants identiques ou différents choisis parmi : -(CH2)_{n'}-V₁-Y₁, halo, nitro, cyano et aryle;
V₁représente -O-, -S- ou une liaison covalente ;
Y₁ représente un radical (C₁-C₆)alkyle éventuellement substitué par un ou plusieurs radicaux halo identiques ou différents ;
n représente un entier de 0 à 6 et n' un entier de 0 à 2 (étant entendu que lorsque n est égal à 0, alors X₁ ne représente pas le radical alkoxy) ;
ou bien R₁ et R₂ forment ensemble, avec l'atome d'azote auquel ils sont rattachés,
- un hétérobicycloalkyle ou un hétérocycloalkyle éventuellement substitué par un ou plusieurs substituants identiques ou différents choisis parmi : hydroxy, (C₁-C₆)alkyle éventuellement substitué par hydroxy, (C₁-C₆)alkoxy-carbonyle, hétérocycloalkyle et -C(O)-NV₁'Y₁' avec V₁' et Y₁' représentant, indépendamment, l'atome d'hydrogène ou un (C₁-C₆)alkyle ; ou
- un radical de formule : 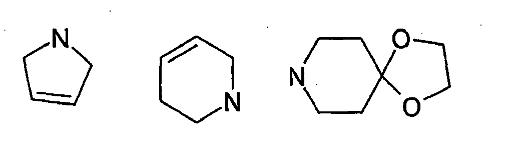
R₃ représente un radical de formule-(CH₂)ₛ-R'₃ ;
R'₃ représente le radical guanidino ; un hétérocycloalkyle contenant au moins un atome d'azote et éventuellement substitué par (C₁-C₆)alkyle ou aralkyle ; un hétéroaryle contenant au moins un atome d'azote et éventuellement substitué par (C₁-C₆)alkyle ; ou un radical de formule -NW₃W'₃
W₃ représente l'atome d'hydrogène ou (C₁-C₈)alkyle ;
W'₃ représente un radical de formule -(CH₂)_{s'}-Z₃ ;
Z₃ représente l'atome d'hydrogène, (C₁-C₈)alkyle éventuellement substitué par un ou plusieurs substituants identiques ou différents choisis parmi : (C₁-C₆)alkoxy, (C₁-C₆)alkylthio et hydroxy ; (C₂-C₆)alkényle ; (C₃-C₇)cycloalkyle éventuellement substitué par un ou plusieurs substituants (C₁-C₆)alkyle identiques ou différents ; cyclohexène ; hétéroaryle et aryle ;
les radicaux aryle et hétéroaryle étant éventuellement substitués par un ou plusieurs radicaux identiques ou différents choisis parmi : le radical de formule -(CH₂)_{s"}-V₃-Y₃, hydroxy, halo, nitro et cyano ;
V₃ représente -O-, -S-, -NH-C(O)-, NV'₃ ou une liaison covalente ;
Y₃ représente un atome d'hydrogène ou un radical (C₁-C₆)alkyle éventuellement substitué par un ou plusieurs radicaux halo identiques ou différents ;
V₃' représente un atome d'hydrogène ou un (C₁-C₆)alkyle ;
s" représente un entier de 0 à 4 ;
ou bien Z₃ représente un radical de formule 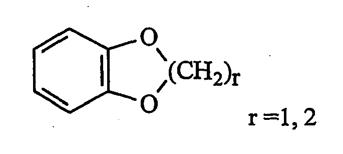
s et s' représentent, indépendamment, un entier de 0 à 6 ;
B représente un radical mono- ou bi-cyclique condensé, insaturé, aromatique ou non-aromatique, contenant éventuellement un ou plusieurs hétéroatomes identiques ou différents choisis parmi O, S et N, et éventuellement substitué par un ou plusieurs radicaux, identiques ou différents, choisis parmi : halo, nitro, cyano, oxy, -X_{B}-Y_{B}, et aryle éventuellement substitué par un ou plusieurs substituants choisis parmi : halo et (C₁-C₆)alkyle éventuellement substitué par un ou plusieurs radicaux halo identiques ou différents ;
X_{B} représente une liaison covalente, -O-, -S-, -C(O)-, -NR_{N}-C(O)-; -C(O)-NR_{N}-, -C(O)-O-, -SO₂- ou -SO₂NH- ;
Y_{B} représente l'atome d'hydrogène ou un radical (C₁-C₆)alkyle éventuellement substitué par un ou plusieurs radicaux halo identiques ou différents ;
R_{N}, représente l'atome d'hydrogène ou un radical (C₁-C₆)alkyle ;
ou un sel pharmaceutiquement acceptable de ces derniers.

2. Composés selon la revendication 1 **caractérisés en ce que** A représente -C(O)- ; ou un sel pharmaceutiquement acceptable de ces derniers.

3. Composés selon l'une des revendications 1 à 2, **caractérisés en ce que** R₁ et R₂ représentent, indépendamment, un radical (C₁-C₈)alkyle; ou un sel pharmaceutiquement acceptable de ces derniers.

4. Composés selon l'une des revendications 1 à 3, **caractérisés en ce que** R₁ et R₂ représentent, indépendamment, un radical (C₁-C₆)alkyle ; ou un sel pharmaceutiquement acceptable de ces derniers.

5. Composés selon l'une des revendications 1 à 4, **caractérisés en ce que** R₁ et R₂ représentent, indépendamment, un radical butyle, pentyle ou isopentyle ; ou un sel pharmaceutiquement acceptable de ces derniers.

6. Composés selon l'une des revendications précédentes, **caractérisés en ce que** R'₃ représente un hétérocycloalkyle contenant au moins un atome d'azote et éventuellement substitué par (C₁-C₆)alkyle ou benzyle ; ou un radical de formule -NW₃W'₃ dans laquelle W₃ représente l'atome d'hydrogène ou un radical (C₁-C₈)alkyle, et W'₃ représente le radical Z₃ et Z₃ représente l'atome d'hydrogène ou un radical (C₁-C₈)alkyle ; ou un sel pharmaceutiquement acceptable de ces derniers.

7. Composés selon l'une des revendications précédentes, **caractérisés en ce que** B représente un radical choisi parmi : phényle, thiényle, pyrrolyle, pyrazolyle, pyridyle, pyrimidyle, pyrazinyle, benzothiényle, thiéno-pyridinyle, thiéno-pyrazinyle, indolyle, benzofuryle, cyclopentènyle, cyclohexenyle, 1,2,3,6-tétrahydropyridinyle, 1,2,3,4-tétrahydropyrimidinyle, 4,5,6,7-tétrahydro-thiéno[2,3-c]pyridinyle, tétrahydrobenzo-thiényle et dihydrocyclopentathiényle.

8. Composés selon la revendication précédente, **caractérisés en ce que** B est éventuellement substitué par un ou plusieurs radicaux, identiques ou différents, choisis parmi : halo, nitro, cyano, oxy, -X_{B}-Y_{B}, et phényle éventuellement substitué par un ou plusieurs substituants choisis parmi : halo et (C₁-C₆)alkyle éventuellement substitué par un ou plusieurs radicaux halo identiques ou différents ;
X_{B} représente une liaison covalente, -O-, -S-, -C(O)-, -NR_{N}-C(O)- ou -C(O)-O- ;
Y_{B} représente l'atome d'hydrogène ou un radical (C₁-C₆)alkyle éventuellement substitué par un ou plusieurs radicaux halo identiques ou différents ;
R_{N} représente l'atome d'hydrogène ; ou un sel pharmaceutiquement acceptable de ces derniers.

9. Composés selon l'une des revendications 1 à 6, **caractérisés en ce que** B représente un radical choisi parmi : phényle, furyle, thiényle, pyrrolyle, pyrazolyle, pyridyle, thiazolyle, pyrazinyle, benzothiényle, thiéno-pyridinyle, thiéno-pyrazinyle, indolyle, benzofuryle, cyclohexenyle, 1,2,3,6-tétrahydropyridinyle, tétrahydrobenzothiényle et dihydrocyclopentathiényle.

10. Composés selon la revendication 9, **caractérisés en ce que** B est éventuellement substitué par un ou plusieurs radicaux, identiques ou différents, choisis parmi : halo, nitro, cyano, -X_{B}-Y_{B}, et phényle éventuellement substitué par un ou plusieurs substituants choisis parmi : halo et (C₁-C₆)alkyle ;
X_{B} représente une liaison covalente, -O-, -S-, -C(O)-, ou -C(O)-O- ;
Y_{B} représente l'atome d'hydrogène ou un radical (C₁-C₆)alkyle ; ou un sel pharmaceutiquement acceptable de ces derniers.

11. Composés selon l'une des revendications 1 à 6, **caractérisés en ce que** B représente un radical choisi parmi : phényle, furyle, thiényle, pyrrolyle, pyrazolyle, pyridyle, pyrazinyle, benzothiényle, thiéno-pyridinyle, indolyle, benzofuryle, cyclohexenyle, 1,2,3,6-tétrahydropyridinyle, tétrahydrobenzothiényle et dihydrocyclopentathiényle.

12. Composés selon la revendication 11, **caractérisés en ce que** B est éventuellement substitué par un ou plusieurs radicaux, identiques ou différents, choisis parmi : halo, nitro et -X_{B}-Y_{B} ; X_{B} représente une liaison covalente, -O-, -C(O)- ou -C(O)-O- ; Y_{B} représente un radical (C₁-C₆)alkyle ; ou un sel pharmaceutiquement acceptable de ces derniers.

13. Composés selon la revendication 12, **caractérisés en ce que** B est éventuellement substitué par un ou plusieurs radicaux, identiques ou différents, choisis parmi : halo, nitro ou -X_{B}-Y_{B} ; X_{B} représente une liaison covalente ou -O- et Y_{B} représente un radical (C₁-C₆)alkyle ; ou un sel pharmaceutiquement acceptable de ces derniers.

14. Composés selon l'une des revendications précédentes, **caractérisés en ce que** R'₃ représente un hétérocycloallcyle contenant au moins un atome d'azote et éventuellement substitué par (C₁-C₆)alkyle ; ou un sel pharmaceutiquement acceptable de ces derniers.

15. Composés selon la revendication 14, **caractérisés en ce que** R'₃ représente le radical pipéridinyle ou pyrrolidinyle ; ou un sel pharmaceutiquement acceptable de ces derniers.

16. Composés selon l'une des revendications 1 à 13, **caractérisés en ce que** R'₃ représente un hétérocycloalkyle de 5 à 6 chaînons et contenant un seul atome d'azote et éventuellement un atome d'oxygène ; ou un radical de formule -NW₃W'₃ dans laquelle W₃ représente l'atome d'hydrogène ou un radical (C₁-C₆)alkyle, W₃ représente le radical Z₃ et Z₃ représente l'atome d'hydrogène ou un radical (C₁-C₆)alkyle ; ou un sel pharmaceutiquement acceptable de ces derniers.

17. Composés selon la revendication 16, **caractérisés en ce que** R'₃ représente le radical pipéridinyle ou pyrrolidinyle et s représente un entier de 2 à 4 ; ou un sel pharmaceutiquement acceptable de ces derniers.

18. Composés selon l'une des revendications 1 à 8 et 14-17, **caractérisés en ce que** B représente un radical choisi parmi : phényle, thiényle, pyrrolyle, pyrazolyle, pyridyle, benzothiényle, thiéno-pyridinyle, thiéno-pyrazinyle, indolyle, benzofuryle, cyclohexenyle, 4,5,6,7-tétrahydro-thiéno[2,3-c]pyridinyle et dihydrocyclopentathiényle.

19. Procédé de préparation d'un composé de formule (I) selon l'une des revendications précédentes, **caractérisé en ce que** l'on traite le composé de formule générale : 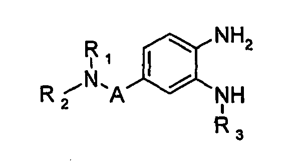 dans laquelle A, R₁, R₂, R₃ ont la signification indiquée ci-dessus, par un isothiocyanate ortho-ester de formule générale 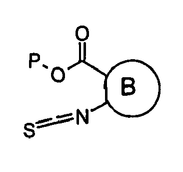 dans laquelle P est un groupement protecteur tel que méthyle, éthyle, *tert*-butyl et B a la signification indiquée ci-dessus en présence d'un agent de couplage ou d'oxyde de mercure (II) jaune en présence de soufre, dans un solvant protique ou aprotique, en présence ou non d'une base.

20. Utilisation d'un composé selon l'une des revendications 1 à 18, pour la préparation d'un médicament pour le traitement des désordres pondéraux, des troubles mentaux, de la douleur ou des désordres de l'activité sexuelle.

21. Utilisation selon la revendication 20, pour le traitement des désordres pondéraux tels que l'obésité, l'anorexie et la cachexie et plus particulièrement la cachexie des pathologies cancéreuses, cachexie du sida, cachexie de la personne âgée, cachexie cardiaque, cachexie rénale, cachexie de l'arthrite rhumatoïde.

22. Utilisation selon la revendication 20, pour le traitement de la douleur et plus particulièrement la douleur neuropathique.

23. Utilisation selon la revendication 20, pour le traitement des troubles mentaux tels que l'anxiété et la dépression.

## Claims

1. Compounds of general formula **(I)** 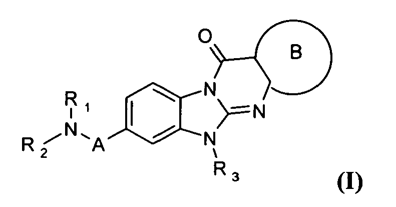 in racemic or enantiomeric form or any combinations of these forms and in which:
A represents -CH₂- or -C(O)- ;
R₁ represents the hydrogen atom; a (C₁-C₈)alkyl radical optionally substituted by hydroxy or one or more identical or different halo radicals; (C₂-C₆)alkenyl; or a radical of formula -(CH₂)ₙ-X₁ ;
R₂ represents a (C₁-C₈)alkyl radical optionally substituted by hydroxy or one or more identical or different halo radicals; (C₂-C₆)alkenyl; or a radical of formula -(CH₂)ₙ-X₁;
each X₁ represents, independently, a (C₁-C₆)alkoxy, (C₃-C₇)cycloalkyl, adamantyl, heterocycloalkyl, aryl or heteroaryl radical,
the (C₃-C₇)cycloalkyl, heterocycloalkyl, aryl and heteroaryl radicals being optionally substituted by one or more identical or different substituents chosen from: -(CH2)_{n'}-V₁-Y₁, halo, nitro, cyano and aryl;
V₁ represents -O-, -S- or a covalent bond;
Y₁ represents a (C₁-C₆)alkyl radical optionally substituted by one or more identical or different halo radicals;
n represents an integer from 0 to 6 and n' an integer from 0 to 2 (it being understood that when n is equal to 0, then X₁ does not represent the alkoxy radical);
or R₁ and R₂ form, together with the nitrogen atom to which they are attached,
- a heterobicycloalkyl or a heterocycloalkyl optionally substituted by one or more identical or different substituents chosen from: hydroxy, (C₁-C₆)alkyl optionally substituted by hydroxy, (C₁-C₆)alkoxy-carbonyl, heterocycloalkyl and -C(O)-NV₁'Y₁' with V₁' and Y₁' representing, independently, the hydrogen atom or a (C₁-C₆)alkyl; or
- a radical of formula: 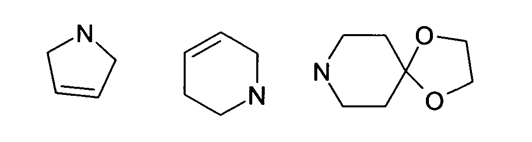
R₃ represents a radical of formula-(CH₂)ₛ-R'₃;
R'₃ represents the guanidino radical; a heterocycloalkyl containing at least one nitrogen atom and optionally substituted by (C₁-C₆)alkyl or aralkyl; a heteroaryl containing at least one nitrogen atom and optionally substituted by (C₁-C₆)alkyl; or a radical of formula -NW₃W'₃
W₃ represents the hydrogen atom or (C₁-C₈)alkyl;
W₃ represents a radical of formula -(CH₂)_{s'}-Z₃;
Z₃ represents the hydrogen atom, (C₁-C₈)alkyl optionally substituted by one or more identical or different substituents chosen from: (C₁-C₆)alkoxy, (C₁-C₆)alkylthio and hydroxy; (C₂-C₆)alkenyl; (C₃-C₇)cycloalkyl optionally substituted by one or more identical or different (C₁-C₆)alkyl substituents; cyclohexene; heteroaryl and aryl;
the aryl and heteroaryl radicals being optionally substituted by one or more identical or different radicals chosen from: the radical of formula -(CH₂)_{s"}-V₃-Y₃, hydroxy, halo, nitro and cyano;
V₃ represents -O-, -S-, -NH-C(O)-, -NV'₃ or a covalent bond;
Y₃ represents a hydrogen atom or a (C₁-C₆)alkyl radical optionally substituted by one or more identical or different halo radicals;
V₃' represents a hydrogen atom or a (C₁-C₆)alkyl;
s" represents an integer from 0 to 4;
or Z₃ represents a radical of formula 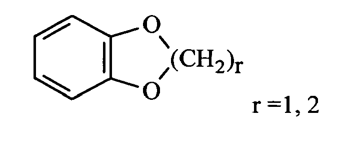
s and s' represent, independently, an integer from 0 to 6;
B represents a condensed, unsaturated, aromatic or non-aromatic mono- or bi-cyclic radical, optionally containing one or more identical or different heteroatoms chosen from O, S and N, and optionally substituted by one or more radicals, identical or different, chosen from: halo, nitro, cyano, oxy, -X_{B}-Y_{B}, and aryl optionally substituted by one or more substituents chosen from: halo and (C₁-C₆)alkyl optionally substituted by one or more identical or different halo radicals;
X_{B} represents a covalent bond, -O-, -S-, -C(O)-, -NR_{N}-C(O)-, -C(O)-NR_{N}-, -C(O)-O-, -SO₂- or -SO₂NH- ;
Y_{B} represents the hydrogen atom or a (C₁-C₆)alkyl radical optionally substituted by one or more identical or different halo radicals;
R_{N} represents the hydrogen atom or a (C₁-C₆)alkyl radical;
or a pharmaceutically acceptable salt thereof.

2. Compounds according to claim 1 **characterized in that** A represents -C(O)-; or a pharmaceutically acceptable salt thereof.

3. Compounds according to one of claims 1 to 2, **characterized in that** R₁ and R₂ represent, independently, a (C₁-C₈)alkyl radical; or a pharmaceutically acceptable salt thereof.

4. Compounds according to one of claims 1 to 3, **characterized in that** R₁ and R₂ represent, independently, a (C₁-C₆)alkyl radical; or a pharmaceutically acceptable salt thereof.

5. Compounds according to one of claims 1 to 4, **characterized in that** R₁ and R₂ represent, independently, a butyl, pentyl or isopentyl radical; or a pharmaceutically acceptable salt thereof.

6. Compounds according to one of the preceding claims, **characterized in that** R'₃ represents a heterocycloalkyl containing at least one nitrogen atom and optionally substituted by (C₁-C₆)alkyl or benzyl; or a radical of formula -NW₃W'₃ in which W₃ represents the hydrogen atom or a (C₁-C₈)alkyl radical, and W'₃ represents the Z₃ radical and Z₃ represents the hydrogen atom or a (C₁-C₈)alkyl radical; or a pharmaceutically acceptable salt thereof.

7. Compounds according to one of the preceding claims, **characterized in that** B represents a radical chosen from: phenyl, thienyl, pyrrolyl, pyrazolyl, pyridyl, pyrimidyl, pyrazinyl, benzothienyl, thieno-pyridinyl, thieno-pyrazinyl, indolyl, benzofuryl, cyclopentenyl, cyclohexenyl, 1,2,3,6-tetrahydropyridinyl, 1,2,3,4-tetrahydropyrimidinyl, 4,5,6,7-tetrahydro-thieno[2,3-c]pyridinyl, tetrahydrobenzo-thienyl and dihydrocyclopentathienyl.

8. Compounds according to the preceding claim, **characterized in that** B is optionally substituted by one or more radicals, identical or different, chosen from: halo, nitro, cyano, oxy, -X_{B}-Y_{B}, and phenyl optionally substituted by one or more substituents chosen from: halo and (C₁-C₆)alkyl optionally substituted by one or more identical or different halo radicals;
X_{B} represents a covalent bond, -O-, -S-, -C(O)-, -NR_{N}-C(O)- or -C(O)-O-;
Y_{B} represents the hydrogen atom or a (C₁-C₆)alkyl radical optionally substituted by one or more identical or different halo radicals;
R_{N} represents the hydrogen atom; or a pharmaceutically acceptable salt thereof.

9. Compounds according to one of claims 1 to 6, **characterized in that** B represents a radical chosen from: phenyl, furyl, thienyl, pyrrolyl, pyrazolyl, pyridyl, thiazolyl, pyrazinyl, benzothienyl, thieno-pyridinyl, thieno-pyrazinyl, indolyl, benzofuryl, cyclohexenyl, 1,2,3,6-tetrahydropyridinyl, tetrahydrobenzothienyl and dihydrocyclopentathienyl.

10. Compounds according to claim 9, **characterized in that** B is optionally substituted by one or more radicals, identical or different, chosen from: halo, nitro, cyano, -X_{B}-Y_{B}, and phenyl optionally substituted by one or more substituents chosen from: halo and (C₁-C₆)alkyl;
X_{B} represents a covalent bond, -O-, -S-, -C(O)-, or -C(O)-O-;
Y_{B} represents the hydrogen atom or a (C₁-C₆)alkyl radical; or a pharmaceutically acceptable salt thereof.

11. Compounds according to one of claims 1 to 6, **characterized in that** B represents a radical chosen from: phenyl, furyl, thienyl, pyrrolyl, pyrazolyl, pyridyl, pyrazinyl, benzothienyl, thieno-pyridinyl, indolyl, benzofuryl, cyclohexenyl, 1,2,3,6-tetrahydropyridinyl, tetrahydrobenzothienyl and dihydrocyclopentathienyl.

12. Compounds according to claim 11, **characterized in that** B is optionally substituted by one or more radicals, identical or different, chosen from: halo, nitro and -X_{B}-Y_{B}; X_{B} represents a covalent bond, -O-, -C(O)- or -C(O)-O-; Y_{B} represents a (C₁-C₆)alkyl radical; or a pharmaceutically acceptable salt thereof.

13. Compounds according to claim 12, **characterized in that** B is optionally substituted by one or more radicals, identical or different, chosen from: halo, nitro or -X_{B}-Y_{B}; X_{B} represents a covalent bond or -O- and Y_{B} represents a (C₁-C₆)alkyl radical; or a pharmaceutically acceptable salt thereof.

14. Compounds according to one of the preceding claims, **characterized in that** R'₃ represents a heterocycloalkyl containing at least one nitrogen atom and optionally substituted by (C₁-C₆)alkyl; or a pharmaceutically acceptable salt thereof.

15. Compounds according to claim 14, **characterized in that** R'₃ represents the piperidinyl or pyrrolidinyl radical; or a pharmaceutically acceptable salt thereof.

16. Compounds according to one of claims 1 to 13, **characterized in that** R'₃ represents a heterocycloalkyl with 5 to 6 members and containing a single nitrogen atom and optionally an oxygen atom; or a radical of formula -NW₃W'₃ in which W₃ represents the hydrogen atom or a (C₁-C₆)alkyl radical, W'₃ represents the Z₃ radical and Z₃ represents the hydrogen atom or a (C₁-C₆)alkyl radical; or a pharmaceutically acceptable salt thereof.

17. Compounds according to claim 16, **characterized in that** R'₃ represents the piperidinyl or pyrrolidinyl radical and s represents an integer from 2 to 4; or a pharmaceutically acceptable salt thereof.

18. Compounds according to one of claims 1 to 8 and 14-17, **characterized in that** B represents a radical chosen from: phenyl, thienyl, pyrrolyl, pyrazolyl, pyridyl, benzothienyl, thieno-pyridinyl, thieno-pyrazinyl, indolyl, benzofuryl, cyclohexenyl, 4,5,6,7-tetrahydro-thieno[2,3-c]pyridinyl and dihydrocyclopentathienyl.

19. Method for the preparation of a compound of formula (I) according to one of the preceding claims, **characterized in that** the compound of general formula: 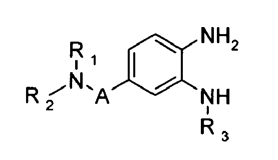 in which A, R₁, R₂, R₃ have the meaning given above, is treated with an ortho-ester isothiocyanate of general formula 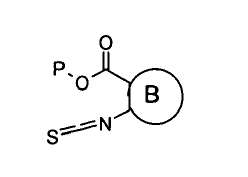 in which P is a protective group such as methyl, ethyl, *tert*-butyl and B has the meaning given above in the presence of a coupling agent or yellow mercury (II) oxide in the presence of sulphur, in a protic or aprotic solvent, in the presence or not of a base.

20. Use of a compound according to one of claims 1 to 18, for the preparation of a medicament for the treatment of weight disorders, mental disorders, pain or sexual activity disorders.

21. Use according to claim 20, for the treatment of weight disorders such as obesity, anorexia and cachexia and more particularly cachexia of cancerous pathologies, AIDS cachexia, cachexia in older persons, cardiac cachexia, renal cachexia, rheumatoid arthritis cachexia.

22. Use according to claim 20, for the treatment of pain and more particularly neuropathic pain.

23. Use according to claim 20, for the treatment of mental disorders such as anxiety and depression.

## Patentansprüche

1. Verbindungen der allgemeinen Formel (I) 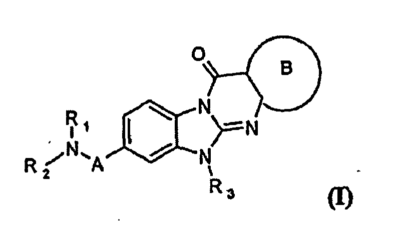 in racemischer Form, in enantiomerer Form oder in allen Kombinationen dieser Formen, in der:
A -CH₂- oder -C(O)- darstellt;
R₁ darstellt: ein Wasserstoffatom; einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel -(CH₂)ₙ-X₁ ;
R₂ darstellt: einen (C₁-C₈)-Alkylrest, der gegebenenfalls mit Hydroxy oder einem oder mehreren gleichen oder verschiedenen Halogenresten substituiert ist; (C₂-C₆)-Alkenyl; oder einen Rest der Formel - (CH₂) ₙ-X₁;
jedes X₁ unabhängig voneinander einen der Reste (C₁-C₆) -Alkoxy, (C₃-C₇)-Cycloalkyl, Adamantyl, Heterocycloalkyl, Aryl oder Heteroaryl darstellt,
wobei die Reste (C₃-C₇)-Cycloalkyl, Heterocycloalkyl, Aryl und Heteroaryl gegebenenfalls mit einem oder mehreren gleichen oder verschiedenen Substituenten substituiert sind, die ausgewählt sind aus: (CH₂)_{n'}-V₁-Y₁, Halogen, Nitro, Cyano und Aryl;
V₁ -O-, -S- oder eine kovalente Bindung darstellt;
Y₁ einen Rest (C₁-C₆) -Alkyl darstellt, der gegebenenfalls mit einem oder mehreren gleichen oder verschiedenen Halogenresten substituiert ist;
n eine ganze Zahl von 0 bis 6 und n' eine ganze Zahl von 0 bis 2 darstellt (wobei gilt, dass, wenn n gleich 0, dann X₁ nicht den Alkoxyrest darstellt);
oder R₁ und R₂ zusammen mit dem Stickstoffatom, an das sie gebunden sind, bilden:
- ein Heterobicycloalkyl oder ein Heterocycloalkyl, das gegebenenfalls mit einem oder mehreren gleichen oder verschiedenen Substituenten substituiert ist, die ausgewählt sind aus: Hydroxy, gegebenenfalls mit Hydroxy substituiertem (C₁-C₆) -Alkyl, (C₁-C₆) - Alkoxycarbonyl, Heterocycloalkyl und -C(O)-NV₁'Y₁', wobei V₁' und Y₁' unabhängig voneinander ein Wasserstoffatom oder ein (C₁-C₆)-Alkyl darstellen; oder
- einen Rest der Formel: 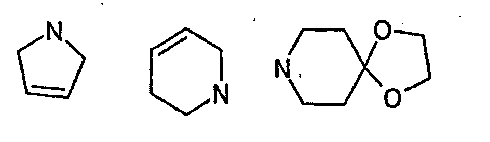
R₃ einen Rest der Formel -(CH₂)ₛ-R'₃ darstellt;
R'₃ darstellt: den Guanidinorest; ein Heterocycloalkyl, das mindestens ein Stickstoffatom enthält und gegebenenfalls mit (C₁-C₆)-Alkyl oder Aralkyl substituiert ist; ein Heteroaryl, das mindestens ein Stickstoffatom enthält und gegebenenfalls mit (C₁-C₆)-Alkyl substituiert ist; oder einen Rest der Formel -NW₃W'₃
W₃ ein Wasserstoffatom oder (C₁-C₈)-Alkyl darstellt;
W'₃ einen Rest der Formel -(CH₂)_{s'}-Z₃ darstellt;
Z₃ darstellt: ein Wasserstoffatom, (C₁-C₈) -Alkyl, das gegebenenfalls mit einem oder mehreren gleichen oder verschiedenen Substituenten substituiert ist, die ausgewählt sind aus: (C₁-C₆) - Alkoxy, (C₁-C₆) -Alkylthio und Hydroxy; (C₂-C₆)-Alkenyl; (C₃-C₇)-Cycloalkyl, das gegebenenfalls mit einem oder mehreren gleichen oder verschiedenen Substituenten (C₁-C₆)-Alkyl substituiert ist; Cyclohexen; Heteroaryl und Aryl;
wobei die Reste Aryl und Heteroaryl gegebenenfalls mit einem oder mehreren gleichen oder verschiedenen Resten substituiert sind, die ausgewählt sind aus: dem Rest der Formel - (CH₂)_{s"} -V₃-Y₃ , Hydroxy, Halogen, Nitro und Cyano;
V₃ -O-, -S-, -NH-C(O)-, -NV'₃ oder eine kovalente Bindung darstellt;
Y₃ ein Wasserstoffatom oder einen (C₁-C₆) - Alkylrest darstellt, der gegebenenfalls mit einem oder mehreren gleichen oder verschiedenen Halogenresten substituiert ist;
V₃' ein Wasserstoffatom oder ein (C₁-C₆) - Alkyl darstellt;
s" eine ganze Zahl von 0 bis 4 darstellt;
oder Z₃ einen Rest der Formel darstellt: 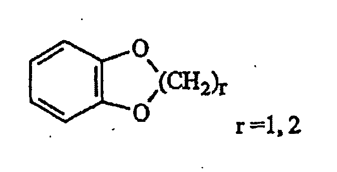
s und s' unabhängig voneinander eine ganze Zahl von 0 bis 6 darstellen;
B einen mono- oder bicyclischen kondensierten, ungesättigten, aromatischen oder nicht aromatischen Rest darstellt, der gegebenenfalls ein oder mehrere gleiche oder verschiedene Heteroatome enthält, die aus O, S und N ausgewählt sind, und gegebenenfalls mit einem oder mehreren gleichen oder verschiedenen Resten substituiert ist, die ausgewählt sind aus: Halogen, Nitro, Cyano, Oxy, -X_{B}-Y_{B} und Aryl, das gegebenenfalls mit einem oder mehreren Substituenten substituiert ist, die ausgewählt sind aus: Halogen und (C₁-C₆)-Alkyl, das gegebenenfalls mit einem oder mehreren gleichen oder verschiedenen Halogenresten substituiert ist;
X_{B} eine kovalente Bindung, -O-, -S-, -C(O)-, -NR_{N}-C(O)-, -C(O)-NR_{N}-, -C(O)-O-, -SO₂- oder -SO₂NH- darstellt;
Y_{B} ein Wasserstoffatom oder einen (C₁-C₆) -Alkyl - rest darstellt, der gegebenenfalls mit einem oder mehreren gleichen oder verschiedenen Halogenresten substituiert ist;
R_{N} ein Wasserstoffatom oder einen (C₁-C₆) -Alkyl - rest darstellt;
oder ein pharmazeutisch annehmbares Salz dieser Verbindungen.

2. Verbindungen nach Anspruch 1, **dadurch gekennzeichnet, dass** A -C(O)- darstellt; oder ein pharmazeutisch annehmbares Salz dieser Verbindungen.

3. Verbindungen nach einem der Ansprüche 1 bis 2, **dadurch gekennzeichnet, dass** R₁ und R₂ unabhängig voneinander einen (C₁-C₈) -Alkylrest darstellen; oder ein pharmazeutisch annehmbares Salz dieser Verbindungen.

4. Verbindungen nach einem der Ansprüche 1 bis 3, **dadurch gekennzeichnet, dass** R₁ und R₂ unabhängig voneinander einen (C₁-C₆)-Alkylrest darstellen; oder ein pharmazeutisch annehmbares Salz dieser Verbindungen.

5. Verbindungen nach einem der Ansprüche 1 bis 4, **dadurch gekennzeichnet, dass** R₁ und R₂ unabhängig voneinander einen Butyl-, Pentyl- oder Isopentylrest darstellen; oder ein pharmazeutisch annehmbares Salz dieser Verbindungen.

6. Verbindungen nach einem der vorhergehenden Ansprüche, **dadurch gekennzeichnet, dass** R'₃ darstellt: ein Heterocycloalkyl, das mindestens ein Stickstoffatom enthält und gegebenenfalls mit (C₁-C₆) -Alkyl oder Benzyl substituiert ist; oder einen Rest der Formel -NW₃W'₃, in der W₃ ein Wasserstoffatom oder einen (C₁-C₈)-Alkylrest darstellt, und W'₃ den Rest Z₃ darstellt und Z₃ ein Wasserstoffatom oder einen (C₁-C₈)-Alkylrest darstellt; oder ein pharmazeutisch annehmbares Salz dieser Verbindungen.

7. Verbindungen nach einem der vorhergehenden Ansprüche, **dadurch gekennzeichnet, dass** B einen Rest darstellt, der ausgewählt ist aus: Phenyl, Thienyl, Pyrrolyl, Pyrazolyl, Pyridyl, Pyrimidyl, Pyrazinyl, Benzothienyl, Thienopyridinyl, Thienopyrazinyl, Indolyl, Benzofuryl, Cyclopentenyl, Cyclohexenyl, 1,2,3,6-Tetrahydropyridinyl, 1,2,3,4-Tetrahydropyrimidinyl, 4,5,6,7-Tetrahydrothieno[2,3-c]pyridinyl, Tetrahydrobenzothienyl und Dihydrocyclopentathienyl.

8. Verbindungen nach dem vorhergehenden Anspruch, **dadurch gekennzeichnet, dass** B gegebenenfalls mit einem oder mehreren gleichen oder verschiedenen Resten substituiert ist, die ausgewählt sind aus: Halogen, Nitro, Cyano, Oxy, -X_{B}-Y_{B} und Phenyl, das gegebenenfalls mit einem oder mehreren Substituenten substituiert ist, die ausgewählt sind aus: Halogen und (C₁-C₆)-Alkyl, das gegebenenfalls mit einem oder mehreren gleichen oder verschiedenen Halogenresten substituiert ist;
X_{B} eine kovalente Bindung, -O-, -S-, -C(O)-, -NR_{N}-C(O)- oder -C (O) -O- darstellt;
Y_{B} ein Wasserstoffatom oder einen (C₁-C₆) -Alkylrest darstellt, der gegebenenfalls mit einem oder mehreren gleichen oder verschiedenen Halogenresten substituiert ist;
R_{N} ein Wasserstoffatom darstellt; oder ein pharmazeutisch annehmbares Salz dieser Verbindungen.

9. Verbindungen nach einem der Ansprüche 1 bis 6, **dadurch gekennzeichnet, dass** B einen Rest darstellt, der ausgewählt ist aus: Phenyl, Furyl, Thienyl, Pyrrolyl, Pyrazolyl, Pyridyl, Thiazolyl, Pyrazinyl, Benzothienyl, Thienopyridinyl, Thienopyrazinyl, Indolyl, Benzofuryl, Cyclohexenyl, 1,2,3,6-Tetrahydropyridinyl, Tetrahydrobenzothienyl und Dihydrocyclopentathienyl.

10. Verbindungen nach Anspruch 9, **dadurch gekennzeichnet, dass** B gegebenenfalls mit einem oder mehreren gleichen oder verschiedenen Resten substituiert ist, die ausgewählt sind aus: Halogen, Nitro, Cyano, -X_{B}-Y_{B} und Phenyl, das gegebenenfalls mit einem oder mehreren Substituenten substituiert ist, die ausgewählt sind aus: Halogen und (C₁-C₆) -Alkyl;
X_{B} eine kovalente Bindung, -O-, -S-, -C(O)- oder -C(O)-O- darstellt;
Y_{B} ein Wasserstoffatom oder einen (C₁-C₆) - Alkylrest darstellt; oder ein pharmazeutisch annehmbares Salz dieser Verbindungen.

11. Verbindungen nach einem der Ansprüche 1 bis 6, **dadurch gekennzeichnet, dass** B einen Rest darstellt, der ausgewählt ist aus Phenyl, Furyl, Thienyl, Pyrrolyl, Pyrazolyl, Pyridyl, Pyrazinyl, Benzothienyl, Thienopyridinyl, Indolyl, Benzofuryl, Cyclohexenyl, 1,2,3,6-Tetrahydropyridinyl, Tetrahydrobenzothienyl und Dihydrocyclopentathienyl.

12. Verbindungen nach Anspruch 11, **dadurch gekennzeichnet, dass** B gegebenenfalls mit einem oder mehreren gleichen oder verschiedenen Resten substituiert ist, die ausgewählt sind aus: Halogen, Nitro und -X_{B}-Y_{B}; X_{B} eine kovalente Bindung, -O-, -C(O)- oder -C(O)-O- darstellt; Y_{B} einen (C₁-C₆) -Alkylrest darstellt; oder ein pharmazeutisch annehmbares Salz dieser Verbindungen.

13. Verbindungen nach Anspruch 12, **dadurch gekennzeichnet, dass** B gegebenenfalls mit einem oder mehreren gleichen oder verschiedenen Resten substituiert ist, die ausgewählt sind aus: Halogen, Nitro oder -X_{B}-Y_{B}; X_{B} eine kovalente Bindung oder -O- darstellt und Y_{B} einen (C₁-C₆)-Alklyrest darstellt; oder ein pharmazeutisch annehmbares Salz dieser Verbindungen.

14. Verbindungen nach einem der vorhergehenden Ansprüche, **dadurch gekennzeichnet, dass** R'₃ ein Heterocycloalkyl darstellt, das mindestens ein Stickstoffatom enthält und gegebenenfalls mit (C₁-C₆)-Alkyl substituiert ist; oder ein pharmazeutisch annehmbares Salz dieser Verbindungen.

15. Verbindungen nach Anspruch 14, **dadurch gekennzeichnet, dass** R'₃ den Piperidinyl- oder Pyrrolidinylrest darstellt; oder ein pharmazeutisch annehmbares Salz dieser Verbindungen.

16. Verbindungen nach einem der Ansprüche 1 bis 13, **dadurch gekennzeichnet, dass** R'₃ darstellt: ein Heterocycloalkyl von 5 bis 6 Gliedern, das ein einziges Stickstoffatom und gegebenenfalls ein Sauerstoffatom enthält; oder einen Rest der Formel -NW₃W'₃, in der W₃ ein Wasserstoffatom oder einen (C₁-C₆)-Alkylrest darstellt, W'₃ den Rest Z₃ darstellt und Z₃ ein Wasserstoffatom oder einen (C₁-C₆)-Alkylrest darstellt; oder ein pharmazeutisch annehmbares Salz dieser Verbindungen.

17. Verbindungen nach Anspruch 16, **dadurch gekennzeichnet, dass** R'₃ einen Piperidinyl- oder Pyrrolidinylrest darstellt und s eine ganze Zahl von 2 bis 4 darstellt; oder ein pharmazeutisch annehmbares Salz dieser Verbindungen.

18. Verbindungen nach einem der Ansprüche 1 bis 8 und 14-17, **dadurch gekennzeichnet, dass** B einen Rest darstellt, der ausgewählt ist aus: Phenyl, Thienyl, Pyrrolyl, Pyrazolyl, Pyridyl, Benzothienyl, Thienopyridinyl, Thienopyrazinyl, Indolyl, Benzofuryl, Cyclohexenyl, 4,5,6,7-Tetrahydrothieno[2,3-c]pyridinyl und Dihydrocyclopentathienyl.

19. Verfahren zur Herstellung einer Verbindung der Formel (I) nach einem der vorhergehenden Ansprüche,
**dadurch gekennzeichnet, dass** man die Verbindung der allgemeinen Formel 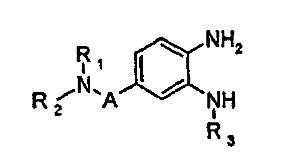 in der A, R₁, R₂, R₃ die oben angegebene Bedeutung haben, mit einem Isothiocyanatorthoester der allgemeinen Formel 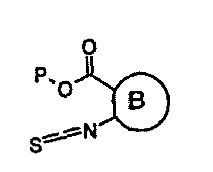 in der P eine Schutzgruppe, wie Methyl, Ethyl, tert-Butyl ist und B die oben angegebene Bedeutung hat, in Gegenwart eines Kopplungsmittels oder von gelbem Quecksilber(II)-oxid in Gegenwart von Schwefel in einem protischen oder aprotischen Lösungsmittel, in Gegenwart oder nicht in Gegenwart einer Base, behandelt.

20. Verwendung einer Verbindung nach einem der Ansprüche 1 bis 18 für die Herstellung eines Medikaments für die Behandlung von Gewichtsstörungen, mentalen Störungen, Schmerz oder Störungen der sexuellen Aktivität.

21. Verwendung nach Anspruch 20 für die Behandlung von Gewichtsstörungen, wie Fettleibigkeit, Anorexie und Kachexie und insbesondere Kachexie bei Krebserkrankungen, Kachexie bei Aids, seniler Kachexie, kardialer Kachexie, renaler Kachexie, Kachexie der rheumatoiden Arthritis.

22. Verwendung nach Anspruch 20 für die Behandlung von Schmerz und insbesondere von neuropathischem Schmerz.

23. Verwendung nach Anspruch 20 für die Behandlung von mentalen Störungen wie Angst und Depression.
